# EUROPEAN PATENT APPLICATION

(11) **EP 4 737 577 A1**
(43) Date of publication of application: **06.05.2026**
(21) Application number: 24831028.6
(22) Date of filing: 28.06.2024
(51) Int. Cl.: C12N 15/85, A01K 67/0278, A61K 49/00, C12N 15/12, C12N 5/10, C12Q 1/02, A01N 1/00, G01N 33/50

(54) **CFB GENE MODIFIED NON-HUMAN ANIMAL**

(30) Priority: 28.06.2023 CN 202310778275; 06.02.2024 CN 202410169335; 17.04.2024 CN 202410462699; 07.05.2024 CN 202410555788
(71) Applicant: Biocytogen Pharmaceuticals (Beijing) Co., Ltd., Beijing 102600 (CN)
(72) Inventor: ZHANG, Shujin, Beijing 102600 (CN); ZHOU, Xiaofei, Beijing 102600 (CN); ZHANG, Zan, Beijing 102600 (CN)
(74) Representative: Fish & Richardson P.C.
(86) International application number: PCT/CN2024/102549
(87) International publication number: WO 2025/002401

(57) **Abstract**

The disclosure relates to a non-human animal that express a human or chimeric (e.g., humanized) CFB protein and methods of use thereof. The disclosure further provides a non-human animal genome, a humanized CFB gene, and cells, tissues, organs, or non-human animals comprising the non-human animal genome or the humanized CFB gene. The non-human animals obtained in present disclosure successfully express human or humanized CFB protein, which can be cleaved by CFD, bind to C3b, and exert functions similar to those in the human. Moreover, the non-human animals obtained in present disclosure maintain intact renal function status and normal blood biochemical parameters. Notably, these non-human animals do not result in the potential pathologies observed in some other transgenic mice known in the art.

## Description

### CROSS-REFERENCES OF RELATED APPLICATIONS

This application claims the benefit of Chinese Patent Application App. No. 202310778275.7, filed on June 28, 2023; Chinese invention Chinese Patent Application App. No. 202410169335.X filed on February 6, 2024; Chinese Patent Application App. No. 202410462699.7, filed on April 17, 2024 and Chinese Patent Application App. No. 202410555788.6 filed on May 7, 2024. The entire contents of the foregoing applications are incorporated herein by reference.

### TECHNICAL FIELD

This disclosure relates to genetically modified animals expressing human or chimeric (e.g., humanized) CFB protein, and methods of use thereof.

### BACKGROUND

The traditional drug research and development typically use *in vitro* screening approaches. However, these screening approaches cannot provide the body environment (such as tumor microenvironment, stromal cells, extracellular matrix components and immune cell interaction, etc.), resulting in a higher rate of failure in drug development. In addition, in view of the differences between humans and animals, the test results obtained from the use of conventional experimental animals for *in vivo* pharmacological test may not reflect the real disease state and the interaction at the targeting sites, resulting in that the results in many clinical trials are significantly different from the animal experimental results.

Therefore, the development of humanized animal models that are suitable for human antibody screening and evaluation will significantly improve the efficiency of new drug development and reduce the cost for drug research and development.

However, the construction of humanized animal models remains highly challenging. For instance, the non-patent literature "Generation and utility of genetically humanized mouse models" (Scheer N, Snaith M, Wolf CR, Seibler J., Drug Discovery Today; 18(23-24):1200-11, 2013) points out that even with meticulously designed strategies, there is no guarantee of achieving proper expression and functionality of the human gene in animals.

### SUMMARY

In view of the deficiencies in the prior art, disclosure is related to a genetically modified non-human animal. This animal expresses human CFB protein normally *in vivo,* which can be cleaved by CFD, binds to C3b, and exerts functions similar to those in humans. Importantly, the non-human animal obtained in this application exhibits intact renal function and normal blood biochemical parameters. Even more notably, it does not develop the potential diseases that are known in the art to occur in certain other transgenic mouse models.

This disclosure is related to an animal model with human or chimeric CFB. The animal model can express human or chimeric CFB (e.g., humanized CFB). It can be used in the studies on the function of CFB genes, and can be used in the screening and evaluation of CFB signaling pathway modulators (e.g., anti-human CFB antibodies, oligonucleotide drugs, and/or polypeptides drugs). In addition, the animal models prepared by the methods described herein can be used in drug screening, pharmacodynamics studies, treatments for immune-related diseases, and diseases therapy for human CFB target site; they can also be used to facilitate the development and design of new drugs, and save time and cost. In summary, this disclosure provides a powerful tool for studying the function of CFB protein and a platform for screening drugs.

In one aspect, the disclosure is related to a genetically-modified, non-human animal whose genome comprises at least one chromosome comprising a sequence encoding a human or chimeric complement factor B (CFB). In some embodiments, the sequence encoding the human or chimeric CFB is regulated by regulatory elements (e.g., an endogenous regulatory element or a human regulatory element; preferably, the regulatory element is the 5'UTR and/or the 3'UTR). In some embodiments, the chimeric CFB is a humanized CFB, and the humanized CFB comprises a portion of a human CFB. In some embodiments, the portion of the human CFB comprises the Ba region and/or the Bb region; preferably, the Bb region comprises a VWA domain; further preferably, the Bb region comprises von Willebrand factor and/or peptidase S1. In some embodiments, the humanized CFB comprises at least 50 to 764 contiguous amino acid sequences that are identical to a portion of the human CFB. In some embodiments, the animal is a mammal, such as a monkey or a rodent (e.g., a mouse or a rat). In some embodiments, the human or chimeric CFB comprises an amino acid sequence set forth in SEQ ID NO: 2 or amino acids 26-764 of SEQ ID NO: 2, or an amino acid sequence that is at least 70%, 75%, 80%, 85%, 90%, 95%, or 99% identical to SEQ ID NO: 2 or amino acids 26-764 of SEQ ID NO: 2, or an amino acid sequence that differs from SEQ ID NO: 2 or amino acids 26-764 of SEQ ID NO: 2 by no more than 10, 9, 8, 7, 6, 5, 4, 3, 2, or 1 amino acid, or an amino acid sequence comprising a substitution, a deletion and/or insertion of one or more amino acid residue set forth in SEQ ID NO: 2 or amino acids 26-764 of SEQ ID NO: 2. In some embodiments, the animal does not express endogenous CFB or expresses a decreased level of endogenous CFB as compared to CFB expression level in a wild-type animal. In some embodiments, the nucleotide sequence encoding the endogenous CFB protein in the non-human animal is silenced or disrupted (e.g., deleted). In some embodiments, the nucleotide sequence encoding the endogenous CFB protein in the non-human animal is deleted, e.g., a nucleotide sequence encoding SEQ ID NO: 1 or amino acids 18-712 thereof is deleted. In some embodiments, the modified CFB gene in the genome of the non-human animal is homozygous or heterozygous with respect to the replacement at the endogenous CFB gene locus. In some embodiments, the human or chimeric CFB expressed by the non-human animal has at least one CFB activity, e.g., non-human animal CFB activity and/or human CFB activity. In some embodiments, the non-human animal has a renal function status substantially consistent with that of a wild-type non-human animal. In some embodiments, compared to a wild-type non-human animal, the levels of urea, serum creatinine, and/or total protein in the non-human animal show no significant difference. In some embodiments, the human or chimeric CFB expressed by the non-human animal can be cleaved by human or non-human animal CFD. In some embodiments, the human or chimeric CFB expressed by the non-human animal can bind to human or non-human animal C3b. In some embodiments, the non-human animal further comprises a nucleotide sequence of a human or chimeric protein encoded by another gene, and the human or chimeric protein includes, but is not limited to, one or more selected from the group consisting of CFD, C3, C5, C5AR1, ANGPTL3, IL36R, IGF1R, TSLP, TLR8, OX40, PD-1, PD-L1, and CTLA4.

In one aspect, the disclosure is related to a genetically-modified, non-human animal, wherein at the endogenous CFB gene locus, the endogenous CFB gene nucleotide sequence is replaced with a nucleotide sequence comprising a nucleotide sequence of the human CFB gene. In some embodiments, the expression of the nucleotide sequence of the human CFB gene is regulated by a regulatory element (e.g., an endogenous regulatory element or a human regulatory element). In some embodiments, the animal has one or more cells expressing human or chimeric CFB. In some embodiments, the animal does not express endogenous CFB or expresses a decreased level of endogenous CFB as compared to CFB expression level in a wild-type animal. In some embodiments, the endogenous CFB is silenced or disrupted (e.g., deleted). In some embodiments, the nucleotide sequence of the human CFB gene comprises a portion of exon 1 to a portion of exon 18 of the human CFB gene; e.g., comprising a portion of exon 1, exon 2, exon 3, exon 4, exon 5, exon 6, exon 7, exon 8, exon 9, exon 10, exon 11, exon 12, exon 13, exon 14, exon 15, exon 16, exon 17, and/or a portion of exon 18; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene. In some embodiments, the nucleotide sequence of human CFB gene further comprises the 5'UTR and/or the 3'UTR of human CFB gene, and further comprises at least a 50 bp to at least 10000 bp contiguous nucleotide sequence upstream of the 5'UTR of human CFB gene and/or at least a 50 bp to at least 5000 bp contiguous nucleotide sequence downstream of the 3'UTR of human CFB gene. In some embodiments, the nucleotide sequence of human CFB gene comprises the 5'UTR of human CFB and at least a 50 bp to at least 10000 bp (e.g., 50, 100, 500, 1000, 2000, 3000, 4000, 5000, 5500, 5600, 5700, 5800, 5900, 6000, 6100, 6200, 7000, 8000, 9000, or 10000 bp, preferably 3000-8000 bp, further preferably 5000-7000 bp) contiguous nucleotide sequence upstream thereof, a nucleotide sequence from the start codon to the stop codon of the human CFB gene, and the 3'UTR of human CFB gene and at least a 50 bp to at least 5000 bp (e.g., 50, 100, 500, 600, 700, 800, 900, 910, 920, 930, 940, 950, 1000, 1100, 1200, 1300, 1400, 1500, 2000, 3000, 4000, or 5000 bp, preferably 500-3000 bp, further preferably 500-1500 bp) contiguous nucleotide sequence downstream thereof. In some embodiments, the nucleotide sequence of human CFB comprises a nucleotide sequence set forth in SEQ ID NO: 7 or 39, or a nucleotide sequence that is at least 70%, 75%, 80%, 85%, 90%, 95%, or 99% identical to SEQ ID NO: 7 or 39, or a nucleotide sequence that differs from SEQ ID NO: 7 or 39 by no more than 50, 40, 30, 20, 10, 9, 6, 3, or 1 nucleotide, or a nucleotide sequence comprising a substitution, a deletion and/or insertion of one or more nucleotides set forth in SEQ ID NO: 7 or 39. In some embodiments, the endogenous CFB gene exons 2 to 17 are replaced; preferably, the endogenous CFB gene further comprises a portion of exon 1, all of intron 1, and/or a portion of intron 17 are replaced; further preferably, the endogenous CFB gene further comprises a portion of exon 18 are replaced. In some embodiments, the endogenous CFB gene is replaced from the start codon to the stop codon thereof, or the endogenous CFB gene is replaced from a portion of exon 1 to a portion of intron 17, wherein the portion of exon 1 comprises up to 70, up to 60, up to 50, up to 40, up to 30, or up to 20 nucleotides at the 3' end of exon 1. In some embodiments, the animal is a mammal, such as a monkey or a rodent (e.g., a mouse or a rat). In some embodiments, the animal is homozygous or heterozygous with respect to the replacement at the endogenous CFB gene locus. In some embodiments, in the genome of the non-human animal, the connection sequence between the upstream of the nucleotide sequence of human CFB and the mouse sequence is SEQ ID NO: 9, and the connection sequence between the downstream of the nucleotide sequence of human CFB and the mouse sequence is SEQ ID NO: 10. In some embodiments, in the genome of the non-human animal, the connection sequence between the upstream of the nucleotide sequence of human CFB and the mouse sequence is SEQ ID NO: 41, and the connection sequence between the downstream of the nucleotide sequence of human CFB and the mouse sequence is SEQ ID NO: 42. In some embodiments, the animal whose genome comprises a modified endogenous CFB nucleotide sequence that transcribes an mRNA comprising SEQ ID NO: 8 or 40, or a nucleotide sequence that is at least 70%, 75%, 80%, 85%, 90%, 95%, or 99% identical to SEQ ID NO: 8 or 40, or a nucleotide sequence that differs from SEQ ID NO: 8 or 40 by no more than 50, 40, 30, 20, 10, 9, 6, 3, or 1 nucleotide, or a nucleotide sequence comprising a substitution, a deletion and/or insertion of one or more nucleotides set forth in SEQ ID NO: 8 or 40. In some embodiments, the animal expresses the human or chimeric CFB having at least one CFB activity, e.g., non-human animal CFB activity and/or human CFB activity. In some embodiments, the non-human animal has a renal function status substantially consistent with that of a wild-type non-human animal. In some embodiments, compared to a wild-type non-human animal, the levels of urea, serum creatinine, and/or total protein in the non-human animal show no significant difference. In some embodiments, the human or chimeric CFB expressed by the non-human animal can be cleaved by human or non-human animal CFD. In some embodiments, the human or chimeric CFB expressed by the non-human animal can bind to human or non-human animal C3b. In some embodiments, the animal further comprises a sequence encoding an additional human or chimeric protein, and the additional human or chimeric protein is one or more selected from the group consisting of CFD, C3, C5, C5AR1, ANGPTL3, IL36R, IGF1R, TSLP, TLR8, OX40, PD-1, PD-L1, and CTLA4.

In one aspect, the disclosure is related to a non-human animal comprising at least one cell comprising a nucleotide sequence encoding a human or humanized CFB polypeptide, wherein the humanized CFB polypeptide comprises at least 50, 100, 200, 300, 400, 500, 600, 700, 710, 720, 730, 740, 750, 760, 763, or 764 contiguous amino acids identical to a human CFB, and the non-human animal expresses the human or humanized CFB. In some embodiments, the nucleotide sequence encoding the human or humanized CFB is operably linked to an endogenous or human CFB regulatory element. In some embodiments, the nucleotide sequence encoding the human or humanized CFB is integrated at the endogenous CFB gene locus of the non-human animal. In some embodiments, the human or humanized CFB has at least one CFB activity, e.g., mouse CFB activity and/or human CFB activity.

In one aspect, the disclosure is related to a non-human animal genome, wherein the animal genome comprises at least one chromosome comprising a sequence encoding a human or chimeric complement factor B (CFB). In some embodiments, the endogenous CFB gene is silenced or disrupted (e.g., deleted). In some embodiments, the endogenous CFB gene is deleted, and the deleted region is replaced with a nucleotide sequence encoding a human or chimeric CFB. In some embodiments, the endogenous CFB gene is deleted, and the deleted region is replaced with a nucleotide sequence of human CFB gene. In some embodiments, the chimeric CFB is a humanized CFB, and the humanized CFB comprises a portion of a human CFB protein and a portion of a non-human animal CFB protein. In some embodiments, the portion of the human CFB comprises the Ba region and/or the Bb region; preferably, the Bb region comprises a VWA domain; further preferably, the Bb region further comprises von Willebrand factor and/or peptidase S1. In some embodiments, the humanized CFB protein comprises at least 50 to 764 contiguous amino acids identical to a portion of the human CFB protein. In some embodiments, the human or chimeric CFB comprises SEQ ID NO: 2, amino acids 26-764 thereof, or an amino acid sequence that is at least 70%, at least 75%, at least 80%, at least 85%, at least 90%, at least 91%, at least 92%, at least 93%, at least 94%, at least 95%, at least 96%, at least 97%, at least 98%, or at least 99% identical to SEQ ID NO: 2 or amino acids 26-764 thereof, or an amino acid sequence that differs from SEQ ID NO: 2 or amino acids 26-764 thereof by no more than 10, 9, 8, 7, 6, 5, 4, 3, 2, or 1 amino acid(s), or an amino acid sequence comprising a substitution, a deletion and/or insertion of one or more amino acid residue in SEQ ID NO: 2 or amino acids 26-764 thereof. In some embodiments, at the endogenous CFB gene locus, the endogenous CFB gene nucleotide sequence is replaced with a nucleotide sequence comprising a nucleotide sequence of the human CFB gene. In some embodiments, the nucleotide sequence of the human CFB gene comprises a portion of exon 1 to a portion of exon 18 of the human CFB gene; wherein the portion of human CFB gene exon 1 comprises at least a 5 bp contiguous nucleotide sequence of human CFB gene exon 1, and the portion of human CFB gene exon 18 comprises at least a 20 bp contiguous nucleotide sequence of human CFB gene exon 18; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene. In some embodiments, the nucleotide sequence of human CFB gene further comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene. In some embodiments, the nucleotide sequence of human CFB gene comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof.

In some embodiments, the nucleotide sequence of human CFB gene comprises a nucleotide sequence set forth in SEQ ID NO: 7 or 39, or comprises a nucleotide sequence that is at least 70%, at least 75%, at least 80%, at least 85%, at least 90%, at least 91%, at least 92%, at least 93%, at least 94%, at least 95%, at least 96%, at least 97%, at least 98%, or at least 99% identical to SEQ ID NO: 7 or 39, or comprises a nucleotide sequence that differs from SEQ ID NO: 7 or 39 by no more than 50, 40, 30, 20, 10, 9, 6, 3, or 1 nucleotide(s), or comprises a nucleotide sequence comprising a substitution, a deletion and/or insertion of one or more nucleotide set forth in SEQ ID NO: 7 or 39. In some embodiments, the deleted endogenous CFB gene comprises a nucleotide sequence encoding an endogenous CFB; preferably, it comprises a nucleotide sequence encoding SEQ ID NO: 1 or amino acids 18-712 thereof. In some embodiments, the endogenous CFB gene exons 2 to 17 are replaced; preferably, the endogenous CFB gene further comprises a portion of exon 1, all of intron 1, and/or a portion of intron 17 are replaced; further preferably, the endogenous CFB gene further comprises a portion of exon 18 are replaced. In some embodiments, the endogenous CFB gene is replaced from the start codon to the stop codon thereof, or the endogenous CFB gene is replaced from a portion of exon 1 to a portion of intron 17, wherein the portion of exon 1 comprises up to 70 nucleotides at the 3' end of exon 1.
In some embodiments, the nucleotide sequence encoding the human or chimeric CFB or the nucleotide sequence of human CFB gene is operably linked to a regulatory element (e.g., an endogenous regulatory element or a human regulatory element) at the CFB gene locus of at least one chromosome. In some embodiments, the non-human animal is a mammal, such as a monkey or a rodent (e.g., a mouse or a rat). In some embodiments, the non-human animal is a mouse. In some embodiments, in the genome of the non-human animal, the connection sequence between the upstream of the nucleotide sequence of human CFB gene and the mouse sequence is SEQ ID NO: 9, and the connection sequence between the downstream of the nucleotide sequence of human CFB and the mouse sequence is SEQ ID NO: 10. In some embodiments, in the genome of the non-human animal, the connection sequence between the upstream of the nucleotide sequence of human CFB and the mouse sequence is SEQ ID NO: 41, and the connection sequence between the downstream of the nucleotide sequence of human CFB and the mouse sequence is SEQ ID NO: 42. In some embodiments, the animal whose genome comprises a modified endogenous CFB nucleotide sequence that transcribes an mRNA comprising SEQ ID NO: 8 or 40, or a nucleotide sequence that is at least 70%, at least 75%, at least 80%, at least 85%, at least 90%, at least 91%, at least 92%, at least 93%, at least 94%, at least 95%, at least 96%, at least 97%, at least 98%, or at least 99% identical to SEQ ID NO: 8 or 40, or a nucleotide sequence that differs from SEQ ID NO: 8 or 40 by no more than 50, 40, 30, 20, 10, 9, 6, 3, or 1 nucleotide, or a nucleotide sequence comprising a substitution, a deletion and/or insertion of one or more nucleotide set forth in SEQ ID NO: 8 or 40.

In one aspect, the disclosure is related to a cell comprising the non-human animal genome described above.

In one aspect, the disclosure is related to a non-human animal comprising the non-human animal genome or the cell described above.

In one aspect, the disclosure is related to a method for making a genetically-modified, non-human animal, at the endogenous CFB gene locus in at least one cell of the non-human animal, the endogenous CFB gene nucleotide sequence is replaced with a nucleotide sequence comprising a nucleotide sequence of the human CFB gene. In some embodiments, the animal does not express endogenous CFB or expresses a decreased level of endogenous CFB as compared to CFB expression level in a wild-type animal. In some embodiments, the nucleotide sequence of the human CFB gene comprises a portion of exon 1 to a portion of exon 18 of the human CFB gene; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene. In some embodiments, the nucleotide sequence of human CFB gene further comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene. In some embodiments, the nucleotide sequence of human CFB gene comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof. In some embodiments, the nucleotide sequence of human CFB comprises a nucleotide sequence set forth in SEQ ID NO: 7 or 39, or a nucleotide sequence that is at least 70%, 75%, 80%, 85%, 90%, 95%, or 99% identical to SEQ ID NO: 7 or 39, or a nucleotide sequence that differs from SEQ ID NO: 7 or 39 by no more than 50, 40, 30, 20, 10, 9, 6, 3, or 1 nucleotide, or a nucleotide sequence comprising a substitution, a deletion and/or insertion of one or more nucleotides set forth in SEQ ID NO: 7 or 39. In some embodiments, the deleted endogenous CFB gene comprises a nucleotide sequence encoding an endogenous CFB; preferably, the deleted endogenous CFB gene comprises a nucleotide sequence encoding SEQ ID NO: 1 or amino acids 18-712 thereof. In some embodiments, the endogenous CFB gene a portion of exons 1 to a portion of 18 are replaced, e.g., comprising exon 1, exon 2, exon 3, exon 4, exon 5, exon 6, exon 7, exon 8, exon 9, exon 10, exon 11, exon 12, exon 13, exon 14, exon 15, exon 16, exon 17, and/or a portion of exon 18; e.g., comprising a nucleotide sequence encoding an endogenous CFB. In some embodiments, the endogenous CFB gene is replaced from a portion of exon 1 to intron 17. In some embodiments, at the CFB gene locus of at least one chromosome, the nucleotide sequence of human CFB gene is operably linked to a regulatory element, e.g., an endogenous regulatory element or a human regulatory element, wherein the regulatory element can be a promoter. In some embodiments, the non-human animal is a mammal, such as a monkey or a rodent (e.g., a mouse or a rat). In some embodiments, the non-human animal is a mouse. In some embodiments, the animal further comprises a sequence encoding an additional human or chimeric protein, and the additional human or chimeric protein is one or more selected from the group consisting of CFD, C3, C5, C5AR1, ANGPTL3, IL36R, IGF1R, TSLP, TLR8, OX40, PD-1, PD-L1, and CTLA4.

In one aspect, the disclosure is related to a method of making a genetically-modified non-human animal cell that expresses a human or chimeric CFB the method comprising: at an endogenous CFB gene locus, the endogenous CFB gene nucleotide sequence is replaced with a nucleotide sequence comprising a nucleotide sequence of the human CFB gene. In some embodiments, the nucleotide sequence of the human CFB gene comprises a portion of exon 1 to a portion of exon 18 of the human CFB gene, e.g., a portion of exon 1, exon 2, exon 3, exon 4, exon 5, exon 6, exon 7, exon 8, exon 9, exon 10, exon 11, exon 12, exon 13, exon 14, exon 15, exon 16, exon 17, and/or a portion of exon 18; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene. In some embodiments, the nucleotide sequence of human CFB gene further comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene. In some embodiments, the nucleotide sequence of human CFB gene comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof. In some embodiments, the expression of the nucleotide sequence of the human CFB gene is regulated by a regulatory element, and the regulatory element is an endogenous regulatory element or a human regulatory element, wherein the regulatory element can be a promoter. In some embodiments, the non-human animal is a mammal, such as a monkey or a rodent (e.g., a mouse or a rat). In some embodiments, the non-human animal is a mouse. In some embodiments, the animal further comprises a sequence encoding an additional human or chimeric protein, and the additional human or chimeric protein is one or more selected from the group consisting of CFD, C3, C5, C5AR1, ANGPTL3, IL36R, IGF1R, TSLP, TLR8, OX40, PD-1, PD-L1, and CTLA4.

In one aspect, the disclosure is related to a humanized CFB gene comprising a nucleotide sequence, wherein the nucleotide sequence is one of the following:
a) a nucleotide sequence encoding SEQ ID NO: 2 or amino acids 26-764 of SEQ ID NO: 2;
b) SEQ ID NO: 3, 4, 5, 6, 7, 8, 9, 10, 11, 12, 37, 38, 39, 40, 41, 42, 43, 44, 45, or 46;
c) a nucleotide sequence having at least 90% identity to SEQ ID NO: 3, 4, 5, 6, 7, 8, 9, 10, 11, 12, 37, 38, 39, 40, 41, 42, 43, 44, 45, or 46;
d) a nucleotide sequence is at least 91%, 92%, 93%, 94%, 95%, 96%, 97%, 98%, or 99% identity to SEQ ID NO: 3, 4, 5, 6, 7, 8, 9, 10, 11, 12, 37, 38, 39, 40, 41, 42, 43, 44, 45, or 46.

In one aspect, the disclosure is related to a polypeptide, wherein the polypeptide is encoded by the humanized CFB gene.

In one aspect, the disclosure is related to a targeting vector, wherein the targeting vector comprises a 5' arm, a donor region, and a 3' arm, wherein the 5' arm is homologous to the 5' end of a region to be altered, the 3' arm is homologous to the 3' end of the region to be altered, and the donor region comprises a nucleotide sequence encoding a human or chimeric CFB. In some embodiments, the region to be altered is located at the endogenous CFB gene locus of a non-human animal; preferably, the region to be altered comprises at least one exon or at least one intron of the endogenous CFB gene of the non-human animal, e.g., a portion of exon 1 to a portion of intron 17 of the endogenous CFB gene, or a portion of exon 1 to a portion of exon 18 of the endogenous CFB gene. In some embodiments, the 5' end of the region to be altered is located in exon 1 of the endogenous CFB gene of the non-human animal, and/or the 3' end of the region to be altered is located in exon 18 of the endogenous CFB gene of the non-human animal. In some embodiments, the 5' end of the region to be altered is located in exon 1 of the endogenous CFB gene of the non-human animal, and/or the 3' end of the region to be altered is located in intron 17 of the endogenous CFB gene of the non-human animal. In some embodiments, the 5' arm sequence is the nucleotide sequence set forth in SEQ ID NO: 3, and the 3' arm sequence is the nucleotide sequence set forth in SEQ ID NO: 4. In some embodiments, the 5' arm sequence is the nucleotide sequence set forth in SEQ ID NO: 5, and the 3' arm sequence is the nucleotide sequence set forth in SEQ ID NO: 6. In some embodiments, the 5' arm sequence is the nucleotide sequence set forth in SEQ ID NO: 37, and the 3' arm sequence is the nucleotide sequence set forth in SEQ ID NO: 38. In some embodiments, the 5' arm sequence is the nucleotide sequence set forth in SEQ ID NO: 45, and the 3' arm sequence is the nucleotide sequence set forth in SEQ ID NO: 46. In some embodiments, the chimeric CFB is a humanized CFB, and the humanized CFB comprises a portion of a human CFB. In some embodiments, the portion of the human CFB comprises the Ba region and/or the Bb region; preferably, the Bb region comprises a VWA domain; further preferably, the Bb region comprises von Willebrand factor and/or peptidase S1. In some embodiments, the chimeric CFB is a humanized CFB, wherein the humanized CFB comprises a portion of a human CFB and a portion of a non-human CFB. In some embodiments, the portion of the human CFB comprises the Ba region and/or the Bb region. Preferably, the Bb region comprises a VWA domain; further preferably, the Bb region comprises von Willebrand factor and/or peptidase S1. In some embodiments, the humanized CFB comprises at least 50 to 764 contiguous amino acid sequences that are identical to a portion of the human CFB. In some embodiments, the human or chimeric CFB comprises an amino acid sequence set forth in SEQ ID NO: 2 or amino acids 26-764 of SEQ ID NO: 2, or an amino acid sequence that is at least 70%, 75%, 80%, 85%, 90%, 95%, or 99% identical to SEQ ID NO: 2 or amino acids 26-764 of SEQ ID NO: 2, or an amino acid sequence that differs from SEQ ID NO: 2 or amino acids 26-764 of SEQ ID NO: 2 by no more than 10, 9, 8, 7, 6, 5, 4, 3, 2, or 1 amino acid, or an amino acid sequence comprising a substitution, a deletion and/or insertion of one or more amino acid residue set forth in SEQ ID NO: 2 or amino acids 26-764 of SEQ ID NO: 2. In some embodiments, the donor region comprises a portion of exon 1 to a portion of exon 18 of the human CFB gene; preferably, the donor region comprises a nucleotide sequence of the coding region of the human CFB gene; further preferably, the donor region comprises a nucleotide sequence from the start codon to the stop codon of the human CFB gene. In some embodiments, the donor region comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene. preferably, the donor region comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof. In some embodiments, the donor region comprises a nucleotide sequence set forth in SEQ ID NO: 7 or 39, or a nucleotide sequence that is at least 70%, 75%, 80%, 85%, 90%, 95%, or 99% identical to SEQ ID NO: 7 or 39, or a nucleotide sequence that differs from SEQ ID NO: 7 or 39 by no more than 50, 40, 30, 20, 10, 9, 6, 3, or 1 nucleotide, or a nucleotide sequence comprising a substitution, a deletion and/or insertion of one or more nucleotides set forth in SEQ ID NO: 7 or 39.

In one aspect, the disclosure is related to a cell, tissue, or organ, wherein the cell, tissue, or organ comprises the humanized CFB gene described above or expresses the polypeptide described above.

In one aspect, the disclosure is related to an animal model, wherein the animal model comprises the humanized CFB gene described above, the polypeptide described above, or the cell, tissue, or organ described above.

In one aspect, the disclosure is related to a use of the non-human animal described above, a non-human animal obtained by the making method described above, the non-human animal genome described above, a cell obtained by the making method described above, the humanized CFB gene described above, the polypeptide described above, the cell, tissue, or organ described above, the animal model described above, or the targeting vector described above, wherein the use comprises: a) use in the development of products related to CFB-associated immune processes involving human cells; b) use as a model system for CFB-associated pharmacological, immunological, microbiological, and medical research; c) use in the production and utilization of animal experimental disease models for CFB-associated etiological studies and/or for the development of diagnostic strategies and/or for the development of therapeutic strategies; d) use in the *in vivo* screening of human CFB signaling pathway modulators, pharmacological efficacy detection, efficacy evaluation, validation, or assessment; or, e) use in the study of CFB gene function, the study of drugs targeting human CFB targets, pharmacological efficacy, and the study of drugs for CFB-associated inflammatory and immune-related diseases.

In one aspect, the disclosure is related to a method of determining the efficacy of a CFB therapeutic agent for treating a disease, wherein the method comprises: 1) administering a CFB therapeutic agent to the non-human animal or animal model of the present disclosure, wherein the non-human animal has a disease; and 2) determining the therapeutic effect of the CFB therapeutic agent on the disease. In some embodiments, the disease is an immune disease, inflammation, or a tumor. In some embodiments, the immune disease is one or more selected from the group consisting of age-related macular degeneration (AMD), rheumatoid arthritis, colitis (including ulcerative colitis or Crohn's disease, e.g., perianal Crohn's disease), rheumatism, multiple sclerosis, Parkinson's disease, asthma, myasthenia gravis, and complement diseases. In some embodiments, the inflammation is one or more selected from the group consisting of IgA nephropathy, C3 glomerulopathy, glomerulonephritis, degenerative inflammation, exudative inflammation (e.g., serous inflammation, fibrinous inflammation, purulent inflammation, hemorrhagic inflammation, necrotizing inflammation, or catarrhal inflammation), proliferative inflammation, and specific inflammation (e.g., tuberculosis, syphilis, leprosy, or lymphogranuloma). In some embodiments, the tumor is one or more selected from the group consisting of solid tumors (e.g., breast cancer) and blood cell tumors (e.g., lymphocyte tumors, B- or T-cell tumors).

In one aspect, the disclosure is related to a method of determining the toxicity of a CFB therapeutic agent, wherein the method comprises: 1) administering a CFB therapeutic agent to the non-human animal or animal model of the present disclosure; and 2) determining the effect of the CFB therapeutic agent on the non-human animal. In some embodiments, determining the effect of the CFB therapeutic agent on the non-human animal involves measuring the body weight of the non-human animal or performing a blood test; preferably, the blood test comprises one or more selected from the group consisting of red blood cell count, hematocrit, and hemoglobin content.

Unless otherwise defined, all technical and scientific terms used herein have the same meaning as commonly understood by one of ordinary skill in the art to which this invention belongs. Methods and materials are described herein for use in the present invention; other, suitable methods and materials known in the art can also be used. The materials, methods, and examples are illustrative only and not intended to be limiting. All publications, patent applications, patents, sequences, database entries, and other references mentioned herein are incorporated by reference in their entirety. In case of conflict, the present specification, including definitions, will control.

In the disclosure, the term "all or a portion," "all" refers to the entirety, and "a portion" refers to a part of the entirety, or some individual components that constitute the whole.

In the disclosure, the term "humanized CFB protein" comprises a portion derived from a human CFB protein, and preferably further comprises a portion of a non-human animal CFB protein. For example, the "human CFB protein" is equivalent to the entirety of a human CFB protein, meaning its amino acid sequence is identical to the full-length amino acid sequence of a human CFB protein. The "portion of a human CFB protein" comprises 5-764 amino acids, which may be consecutive or spaced, and preferably 10-764 or 50-764 consecutive or spaced amino acids, e.g., 5, 10, 20, 30, 40, 50, 60, 70, 80, 90, 100, 200, 300, 400, 500, 600, 700, 730, 735, 739, 750, 760, or 764 amino acids, that are identical to the amino acid sequence of a human CFB protein.

In the disclosure, the term "humanized CFB gene" comprises a portion derived from a human CFB gene and a portion of a non-human animal CFB gene. For example, the "human CFB gene" is equivalent to the entirety of a human CFB gene, meaning its nucleotide sequence is identical to the full-length nucleotide sequence of a human CFB gene. The "portion of a human CFB gene" comprises a nucleotide sequence of 20-5990, or 20-2476, or 20-2295 base pairs (bp), which may be consecutive or spaced, that is identical to the nucleotide sequence of a human CFB gene, e.g., 20, 50, 100, 200, 500, 1000, 2000, 2200, 2250, 2290, 2295, 2300, 2400, 2470, 2475, 2476, 2500, 3000, 3500, 4000, 4500, 5000, 5500, 5900, or 5990 bp that are identical to the nucleotide sequence of a human CFB gene.

In the disclosure, the term "gene locus," broadly speaking, refers to the position a gene occupies on a chromosome; narrowly speaking, it refers to a segment of DNA within a gene, which can be an entire gene or a part of a gene. For example, the "CFB gene locus" refers to any selected DNA fragment within exons 1-18 of the CFB gene. In some embodiments, the replaced endogenous CFB gene locus of the non-human animal can be any selected DNA fragment within exons 1-18 of the endogenous CFB gene of the non-human animal.

In the disclosure, the term "a portion of an exon" means that a nucleotide sequence, consisting of several, tens, or hundreds of nucleotides that may be consecutive or spaced, is identical to the full nucleotide sequence of the exon. For example, a portion of exon 1 of the human CFB gene includes 5-191 bp, preferably 50-100 bp, which may be consecutive or spaced, e.g., 5, 10, 15, 20, 25, 30, 35, 40, 45, 50, 55, 60, 61, 62, 63, 64, 65, 66, 67, 68, 69, 70, 75, 80, 85, 90, 95, 100, 110, 120, 130, 140, 150, 160, 170, 180, 190, or 191 bp, that are identical to the nucleotide sequence of exon 1 of the human CFB gene.

In the disclosure, the term "exon XX to exon XXX," "exon XX-XXX," or "all of exon XX to exon XXX" refers to the inclusion of the specified exons and the introns between them. For example, exon 1 to exon 18 includes the full nucleotide sequences of exon 1, intron 1, exon 2, intron 2, exon 3, intron 3, exon 4, intron 4, exon 5, intron 5, exon 6, intron 6, exon 7, intron 7, exon 8, intron 8, exon 9, intron 9, exon 10, intron 10, exon 11, intron 11, exon 12, intron 12, exon 13, intron 13, exon 14, intron 14, exon 15, intron 15, exon 16, intron 16, exon 17, intron 17, and exon 18. Another example, "a portion of exon XX to a portion of exon XXX" or "a portion of exon XX - a portion of exon XXX" includes a portion of exon 1, intron 1, exon 2, intron 2, exon 3, intron 3, exon 4, intron 4, exon 5, intron 5, exon 6, intron 6, exon 7, intron 7, exon 8, intron 8, exon 9, intron 9, exon 10, intron 10, exon 11, intron 11, exon 12, intron 12, exon 13, intron 13, exon 14, intron 14, exon 15, intron 15, exon 16, intron 16, exon 17, intron 17, and a portion of exon 18.

In the present disclosure, the term "intron xx" refers to the intron between two exons. For example, intron 1 is the intron between exon 1 and exon 2, and intron 17 is the intron between exon 17 and exon 18.

In the disclosure, the term "comprise" or "comprising" is an open-ended expression, meaning it includes the specified component or step described, as well as other components or steps that do not materially affect it. When used to describe the sequence of a protein or nucleic acid, the protein or nucleic acid may consist of said sequence, or it may have additional amino acids or nucleotides at one or both ends, while still possessing the same or similar activity as the original sequence.

In the disclosure, the term "and/or" encompasses all combinations of the items connected by this term, and each combination should be considered as having been individually listed in the disclosure. For example, "A and/or B" includes "A," "A and B," and "B." Another example, "A, B and/or C" includes "A," "B," "C," "A and B," "A and C," "B and C," and "A and B and C."

Other features and advantages of the invention will be apparent from the following detailed description and figures, and from the claims.

### CFB

Complement factor B (CFB) is a single-chain protein. Upon activation of the alternative complement pathway, CFB is cleaved by CFD into the Ba domain (~33 kDa) and the Bb domain (~60 kDa). The Ba domain (residues 1-259) contains three complement control protein (CCP) domains, which are important for the initial binding of CFB to C3b. The Bb domain (residues 260-764) contains a von Willebrand factor A-type domain (VWA) and a peptidase S1 (PA clan) domain. The VWA domain, comprising approximately 200 residues, forms an α/β open-sheet structure. The MIDAS site within the VWA domain is also crucial for the protein's binding to C3b.

CFD is a key regulator of the alternative complement pathway. It acts on the C3b/CFB complex, cleaving CFB to release Ba, while Bb remains attached to C3b. This C3bBb complex forms the C3 convertase. Direct biological effects of Ba and Bb have been reported, although the physiological significance of these effects remains unclear. Ba may exhibit chemotactic activity for neutrophils and macrophages and may potentially limit B-cell proliferation. Bb has been reported to influence macrophage and monocyte function and may promote B-cell growth.

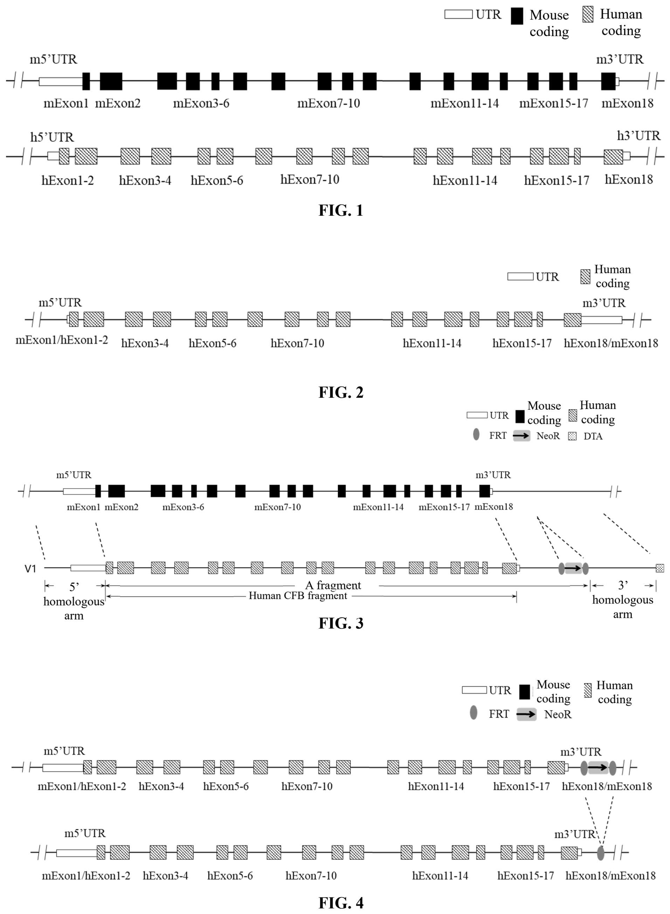

In the human genome, the CFB gene (Gene ID: 629) comprises eighteen exons, exon 1, exon 2, exon 3, exon 4, exon 5, exon 6, exon 7, exon 8, exon 9, exon 10, exon 11, exon 12, exon 13, exon 14, exon 15, exon 16, exon 17, and exon 18 (**FIG. 1**). The nucleotide sequence of human CFB mRNA is NM_001710.6, and the amino acid sequence of human CFB is NP_001701.2 (SEQ ID NO: 2). Based on the transcript NM_001710.6 and its encoded protein NP_001701.2, the positions of each exon within the nucleotide and amino acid sequences are shown in Table 1.

**Table 1**

| **Human CFB (approximate location)** | **NM_001710.6 2476bp** | **NP_001701.2 764aa (SEQ ID NO: 2)** |
|---|---|---|
| Exon 1 | 1-191 | 1-21 |
| Exon 2 | 192-425 | 22-99 |
| Exon 3 | 426-611 | 100-161 |
| Exon 4 | 612-785 | 162-219 |
| Exon 5 | 786-887 | 220-253 |
| Exon 6 | 888-1024 | 254-299 |
| Exon 7 | 1025-1163 | 300-345 |
| Exon 8 | 1164-1295 | 346-389 |
| Exon 9 | 1296-1397 | 390-423 |
| Exon 10 | 1398-1535 | 424-469 |
| Exon 11 | 1536-1633 | 470-502 |
| Exon 12 | 1634-1751 | 503-541 |
| Exon 13 | 1752-1905 | 542-593 |
| Exon 14 | 1906-1982 | 594-618 |
| Exon 15 | 1983-2083 | 619-652 |
| Exon 16 | 2084-2216 | 653-696 |
| Exon 17 | 2217-2266 | 697-713 |
| Exon 18 | 2267-2476 | 714-764 |
| Signal peptide | 128-202 | 1-25 |
| Chain | 203-2419 | 26-764 |
| Donor region in Example 1 | 128-2422 | 1-764 |
| Donor region in Example 2 | 1-2476 | 1-764 |

The human CFB gene (NCBI Gene ID: 629) is located from 31946095 to 31952084 on chromosome 6 in NC_000006.12 (based on transcript NM_001710.6). Within this region, the 5'UTR is from 31946095 to 31946221, exon 1 is from 31,946,095 to 31,946,285, intron 1 is from 31,946,286 to 31,946,372, exon 2 is from 31,946,373 to 31,946,606, intron 2 is from 31,946,607 to 31,947,006, exon 3 is from 31,947,007 to 31,947,192, intron 3 is from 31,947,193 to 31,947,347, exon 4 is from 31,947,348 to 31,947,521, intron 4 is from 31,947,522 to 31,947,741, exon 5 is from 31,947,742 to 31,947,843, intron 5 is from 31,947,844 to 31,947,944, exon 6 is from 31,947,945 to 31,948,081, intron 6 is from 31,948,082 to 31,948,373, exon 7 is from 31,948,374 to 31,948,512, intron 7 is from 31,948,513 to 31,948,829, exon 8 is from 31,948,830 to 31,948,961, intron 8 is from 31,948,962 to 31,949,242, exon 9 is from 31,949,243 to 31,949,344, intron 9 is from 31,949,345 to 31,949,419, exon 10 is from 31,949,420 to 31,949,557, intron 10 is from 31,949,558 to 31,950,049, exon 11 is from 31,950,050 to 31,950,147, intron 11 is from 31,950,148 to 31,950,285, exon 12 is from 31,950,286 to 31,950,403, intron 12 is from 31,950,404 to 31,950,618, exon 13 is from 31,950,619 to 31,950,772, intron 13 is from 31,950,773 to 31,950,867, exon 14 is from 31,950,868 to 31,950,944, intron 14 is from 31,950,945 to 31,951,143, exon 15 is from 31,951,144 to 31,951,244, intron 15 is from 31,951,245 to 31,951,340, exon 16 is from 31,951,341 to 31,951,473, intron 16 is from 31,951,474 to 31,951,554, exon 17 is from 31,951,555 to 31,951,604, intron 17 is from 31,951,605 to 31,951,874, exon 18 is from 31,951,875 to 31,952,084, and the 3'UTR is from 31952031 to 31952084. All the described herein information regarding the human CFB gene locus can be accessed on the NCBI website (Gene ID: 629). Its entire content is incorporated herein by reference.

In the mouse genome, the CFB gene (Gene ID: 14962) comprises eighteen exons, exon 1, exon 2, exon 3, exon 4, exon 5, exon 6, exon 7, exon 8, exon 9, exon 10, exon 11, exon 12, exon 13, exon 14, exon 15, exon 16, exon 17, and exon 18 (**FIG. 1**). The nucleotide sequence of mouse CFB mRNA is NM_008198.2, and the amino acid sequence of mouse CFB is NP_032224.2 (SEQ ID NO: 1). Based on the transcript NM_008198.2 and its encoded protein NP_032224.2, the positions of each exon within the nucleotide and amino acid sequences are shown in Table 2.

**Table 2**

| **Mouse CFB (approximate location)** | **NM_008198.2 2767bp** | **NP_032224.2 763aa (SEQ ID NO: 1)** |
|---|---|---|
| Exon 1 | 1-463 | 1-20 |
| Exon 2 | 464-697 | 21-98 |
| Exon 3 | 698-883 | 99-160 |
| Exon 4 | 884-1057 | 161-218 |
| Exon 5 | 1058-1159 | 219-252 |
| Exon 6 | 1160-1296 | 253-298 |
| Exon 7 | 1297-1435 | 299-344 |
| Exon 8 | 1436-1567 | 345-388 |
| Exon 9 | 1568-1669 | 389-422 |
| Exon 10 | 1670-1807 | 423-468 |
| Exon 11 | 1808-1905 | 469-501 |
| Exon 12 | 1906-2023 | 502-540 |
| Exon 13 | 2024-2177 | 541-592 |
| Exon 14 | 2178-2254 | 593-617 |
| Exon 15 | 2255-2355 | 618-651 |
| Exon 16 | 2356-2488 | 652-695 |
| Exon 17 | 2489-2538 | 696-712 |
| Exon 18 | 2539-2747 | 713-763 |
| Signal peptide | 403-474 | 1-24 |
| Chain | 475-2691 | 25-763 |
| Replaced region in Example 1 | 403-2694 | 1-763 |
| Replaced region in Example 2 | 453-2538 | 18-712 |

The mouse CFB gene (NCBI Gene ID: 14962) is located from 35075350 to 35081492 on chromosome 17 in NC_000083.7 (based on transcript NM_008198.2). Within this region, the 5'UTR is from 35081490 to 35081089, exon 1 is from 35,081,490 to 35,081,028, intron 1 is from 35,081,027 to 35,080,941, exon 2 is from 35,080,940 to 35,080,707, intron 2 is from 35,080,706 to 35,080,190, exon 3 is from 35,080,189 to 35,080,004, intron 3 is from 35,080,003 to 35,079,913, exon 4 is from 35,079,912 to 35,079,739, intron 4 is from 35,079,738 to 35,079,602, exon 5 is from 35,079,601 to 35,079,500, intron 5 is from 35,079,499 to 35,079,399, exon 6 is from 35,079,398 to 35,079,262, intron 6 is from 35,079,261 to 35,079,051, exon 7 is from 35,079,050 to 35,078,912, intron 7 is from 35,078,911 to 35,078,550, exon 8 is from 35,078,549 to 35,078,418, intron 8 is from 35,078,417 to 35,078,308, exon 9 is from 35,078,307 to 35,078,206, intron 9 is from 35,078,205 to 35,078,123, exon 10 is from 35,078,122 to 35,077,985, intron 10 is from 35,077,984 to 35,077,572, exon 11 is from 35,077,571 to 35,077,474, intron 11 is from 35,077,473 to 35,077,131, exon 12 is from 35,077,130 to 35,077,013, intron 12 is from 35,077,012 to 35,076,798, exon 13 is from 35,076,797 to 35,076,644, intron 13 is from 35,076,643 to 35,076,545, exon 14 is from 35,076,544 to 35,076,468, intron 14 is from 35,076,467 to 35,076,296, exon 15 is from 35,076,295 to 35,076,195, intron 15 is from 35,076,194 to 35,076,113, exon 16 is from 35,076,112 to 35,075,980, intron 16 is from 35,075,979 to 35,075,906, exon 17 is from 35,075,905 to 35,075,856, intron 17 is from 35,075,855 to 35,075,559, exon 18 is from 35,075,558 to 35,075,360, and the 3'UTR is from 35075402 to 35075360. All the described herein information regarding the mouse CFB gene locus can be accessed on the NCBI website (Gene ID: 14962). Its entire content is incorporated herein by reference.

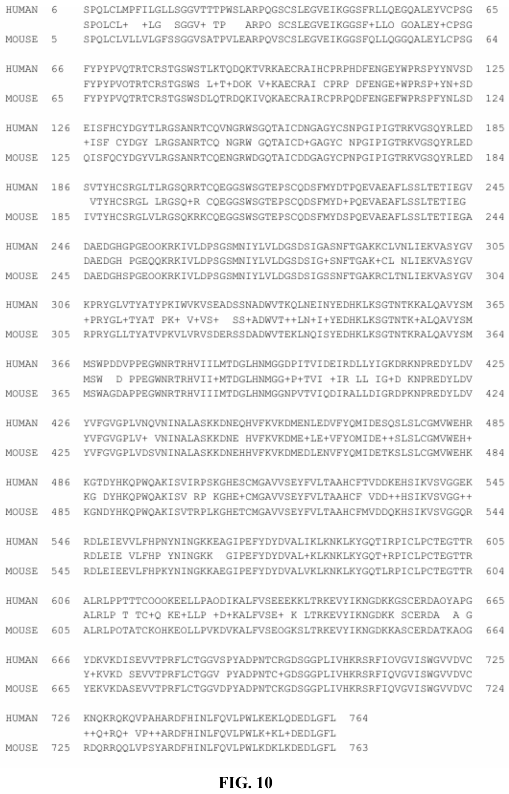

**FIG. 10** and Table 3 show an alignment of the human CFB amino acid sequence (NP_001701.2; SEQ ID NO: 2) and the mouse CFB amino acid sequence (NP_032224.2; SEQ ID NO: 1). Therefore, corresponding amino acid residues or regions between human and mouse CFB can be identified in **FIG. 10****.**

**Table 3**

| Score | Method | Identities | Positives | Gaps |
|---|---|---|---|---|
| 1363 bits(3528) | Compositional matrix adjust. | 639/759(84%) | 700/759(92%) | 0/759(0%) |

CFB genes, proteins, and locus of the other species are also known in the art. For example, the gene ID for CFB in *Rattus norvegicus* (rat) is 294257, the gene ID for CFB in *Macaca mulatta* (Rhesus monkey) is 716809, the gene ID for CFB in Canis lupus familiaris (dog) is 100688056, and the gene ID for CFB in *Sus scrofa* (pig) is 100124383. The relevant information for these genes (e.g., intron sequences, exon sequences, amino acid residues of these proteins) can be found, e.g., in NCBI database, which is incorporated by reference herein in its entirety.

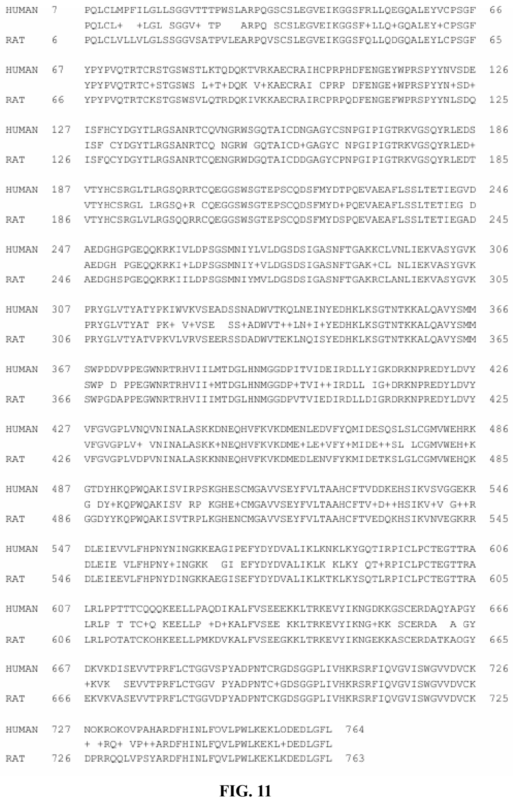

**FIG. 11** and Table 4 show an alignment of the human CFB amino acid sequence (NP_001701.2; SEQ ID NO: 2) and the rat CFB amino acid sequence (NP_997631.2; SEQ ID NO: 57). Therefore, corresponding amino acid residues or regions between human and rat CFB can be identified in **FIG. 11****.**

**Table 4**

| Score | Method | Identities | Positives | Gaps |
|---|---|---|---|---|
| 1343 bits(3475) | Compositional matrix adjust. | 642/758(85%) | 704/758(92%) | 0/758(0%) |

The disclosure is related to a human or chimeric (e.g., humanized) CFB , or a human or chimeric (e.g., humanized) CFB gene.

In some embodiments, the humanized CFB comprises a portion of a human CFB. Preferably, it further comprises a portion of a non-human animal CFB. The portion of the human CFB comprises the Ba region and/or the Bb region. Preferably, the Bb region comprises a VWA domain; further preferably, it further comprises von Willebrand factor and/or peptidase S1. In some embodiments, the humanized CFB comprises the Ba region of a human CFB and the Bb region of a non-human animal CFB. In some embodiments, the humanized CFB protein comprises the Bb region of a human CFB and the Ba region of a non-human animal CFB. In some embodiments, the humanized CFB comprises the VWA domain of a human CFB and the von Willebrand factor, peptidase S1, and Ba region of a non-human animal CFB. In some embodiments, the humanized CFB protein comprises replacing the VWA domain of a non-human animal CFB with the VWA domain region of a human CFB. In some embodiments, the humanized CFB comprises replacing the Bb region of a non-human animal CFB with the Bb region of a human CFB. In some embodiments, the humanized CFB comprises replacing the Ba region of a non-human animal CFB with the Ba region of a human CFB. In some embodiments, the humanized CFB protein comprises at least 50 to 764 (e.g., 50, 60, 70, 80, 90, 100, 150, 200, 220, 240, 260, 280, 300, 310, 350, 400, 450, 500, 550, 600, 650, 700, 710, 720, 730, 739, 740, 750, 760, 761, 762, 763, or 764) contiguous amino acids identical to a portion of a human CFB protein. In some embodiments, the humanized CFB protein comprises SEQ ID NO: 2, amino acids 26-764 thereof, or an amino acid sequence that is at least 70%, at least 75%, at least 80%, at least 85%, at least 90%, at least 91%, at least 92%, at least 93%, at least 94%, at least 95%, at least 96%, at least 97%, at least 98%, or at least 99% identical to SEQ ID NO: 2 or amino acids 26-764 thereof, or an amino acid sequence that differs from SEQ ID NO: 2 or amino acids 26-764 thereof by no more than 10, 9, 8, 7, 6, 5, 4, 3, 2, or 1 amino acid(s), or an amino acid sequence comprising a substitution, a deletion and/or insertion of one or more amino acid residue(s) in SEQ ID NO: 2 or amino acids 26-764 thereof.

In some embodiments, the humanized CFB gene encodes the humanized CFB protein described above. Preferably, it comprises a portion of an endogenous CFB gene of a non-human animal and a portion of a human CFB gene. In some embodiments, the portion of the human CFB gene comprises a nucleotide sequence encoding the Ba region and/or the Bb region of a human CFB. In some embodiments, the portion of the human CFB gene comprises a nucleotide sequence encoding the VWA domain of a human CFB. In some embodiments, the humanized CFB gene comprises, at the endogenous CFB gene locus of the non-human animal, replacing the endogenous CFB gene with a nucleotide sequence comprising human CFB. In some embodiments, the non-human animal is mouse. In some embodiments, the nucleotide sequence of human CFB gene comprises a nucleotide sequence encoding the Ba region and/or the Bb region. In some embodiments, the nucleotide sequence of human CFB gene comprises a nucleotide sequence encoding the VWA domain. In some embodiments, the nucleotide sequence of human CFB gene is genomic sequence, a CDS sequence, or a cDNA sequence. In some embodiments, the nucleotide sequence of human CFB comprises at least one exon and/or at least one intron of the human CFB gene. In some embodiments, the nucleotide sequence of human CFB comprises any one or more of exon 1, exon 2, exon 3, exon 4, exon 5, exon 6, exon 7, exon 8, exon 9, exon 10, exon 11, exon 12, exon 13, exon 14, exon 15, exon 16, exon 17, or exon 18 of the human CFB gene. In some embodiments, the nucleotide sequence of human CFB comprises a portion of exon 1 to a portion of exon 18 of the human CFB gene, wherein the portion of exon 1 comprises a nucleotide sequence of the coding region within exon 1, and the portion of exon 18 comprises a nucleotide sequence of the coding region within exon 18. Preferably, the nucleotide sequence of human CFB comprises a nucleotide sequence of the coding region of the human CFB gene; further preferably, it comprises a nucleotide sequence from the start codon to the stop codon of the human CFB gene. In some embodiments, the nucleotide sequence of human CFB comprises a nucleotide sequence from the start codon to the stop codon of the human CFB gene, preferably further comprising the 5'UTR and/or the 3'UTR of human CFB; further comprising at least a 50 bp contiguous nucleotide sequence upstream of the 5'UTR of human CFB and/or at least a 50 bp contiguous nucleotide sequence downstream of the 3'UTR of human CFB. In some embodiments, the nucleotide sequence of human CFB comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof. In some embodiments, the amino acid sequence of human CFB comprises SEQ ID NO: 2, amino acids 26-764 thereof, or an amino acid sequence that is at least 70%, at least 75%, at least 80%, at least 85%, at least 90%, at least 91%, at least 92%, at least 93%, at least 94%, at least 95%, at least 96%, at least 97%, at least 98%, or at least 99% identical to SEQ ID NO: 2 or amino acids 26-764 thereof, or an amino acid sequence that differs from SEQ ID NO: 2 or amino acids 26-764 thereof by no more than 10, 9, 8, 7, 6, 5, 4, 3, 2, or 1 amino acid(s), or an amino acid sequence comprising a substitution, a deletion and/or insertion of one or more amino acid residue(s) in SEQ ID NO: 2 or amino acids 26-764 thereof. In some embodiments, the nucleotide sequence of human CFB comprises the nucleotide sequence set forth in SEQ ID NO: 7 or 39, or comprises a nucleotide sequence that is at least 70%, at least 75%, at least 80%, at least 85%, at least 90%, at least 91%, at least 92%, at least 93%, at least 94%, at least 95%, at least 96%, at least 97%, at least 98%, or at least 99% identical to SEQ ID NO: 7 or 39, or comprises a nucleotide sequence that differs from SEQ ID NO: 7 or 39 by no more than 50, 40, 30, 20, 10, 9, 6, 3, or 1 nucleotide(s), or comprises a nucleotide sequence comprising a substitution, a deletion and/or insertion of one or more nucleotide(s) set forth in SEQ ID NO: 7 or 39. In some embodiments, the nucleotide sequence encoding the Ba region and/or the Bb region of the endogenous CFB is replaced. In some embodiments, the nucleotide sequence encoding the von Willebrand factor and/or peptidase S1 region of the endogenous CFB is replaced. In some embodiments, the nucleotide sequence encoding SEQ ID NO: 1 or amino acids 18-712 thereof, or SEQ ID NO: 57 is replaced. In some embodiments, at least one exon and/or at least one intron of the endogenous CFB gene is replaced. In some embodiments, one or more of exon 1, exon 2, exon 3, exon 4, exon 5, exon 6, exon 7, exon 8, exon 9, exon 10, exon 11, exon 12, exon 13, exon 14, exon 15, exon 16, exon 17, or exon 18 of the endogenous CFB gene is replaced. In some embodiments, exons 2 to 17 of the endogenous CFB gene are replaced; preferably, the replaced endogenous CFB gene further comprises a portion of exon 1, all of intron 1, and/or a portion of intron 17; further preferably, the replaced endogenous CFB gene further comprises a portion of exon 18. In some embodiments, a portion of exon 1 to a portion of exon 18 of the endogenous CFB gene, e.g., a nucleotide sequence comprising the coding region or from the start codon to the stop codon, is replaced. In some embodiments, a portion of exon 1 to a portion of intron 17 of the endogenous CFB gene is replaced. In some embodiments, the non-human animal is a mammal, such as a monkey or a rodent (e.g., a mouse or a rat). In some embodiments, the entirety of the nucleotide sequences of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the non-human animal (e.g., mouse) CFB gene is replaced with the nucleotide sequence of human CFB. In some embodiments, a "portion" of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the non-human animal (e.g., mouse) CFB gene is replaced with a nucleotide sequence or an amino acid sequence of human CFB. The "portion" refers to at least 1 bp to 2767 bp, or at least 1 bp to at least 2449 bp, e.g., at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 250, 300, 350, 400, 450, 500, 550, 600, 650, 700, 750, 800, 850, 900, 910, 911, 912, 913, 914, 915, 916, 917, 918, 919, 920, 930, 940, 950, 1000, 1100, 1200, 1300, 1400, 1500, 1600, 1750, 1800, 1900, 2000, 2080, 2085, 2100, 2200, 2292, 2295, 2449, 2700, or 2767 bp of contiguous nucleotide sequence from the non-human animal (e.g., mouse) CFB gene, or at least 1 amino acid to 763 amino acids, e.g., at least 1, 2, 3, 4, 5, 6, 7, 8, 9, 10, 20, 30, 40, 50, 60, 70, 80, 90, 100, 150, 200, 220, 240, 260, 280, 300, 310, 350, 400, 450, 500, 550, 600, 650, 690, 695, 700, 710, 720, 730, 740, 750, 760, 761, 762, or 763 contiguous amino acids from the non-human animal (e.g., mouse) CFB protein. In some embodiments, the "portion" has at least 50%, at least 55%, at least 60%, at least 65%, at least 70%, at least 75%, at least 80%, at least 85%, at least 90%, at least 95%, or 100% identity to exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the non-human animal (e.g., mouse) CFB gene. In some embodiments, a "portion" or the "entirety" of the sequences of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the non-human animal (e.g., mouse) CFB gene (e.g., a portion of exon 1, all of exons 2-17, and a portion of exon 18) is replaced with a "portion" or the "entirety" of the sequences of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the human CFB gene (e.g., from a portion of exon 1 to a portion of exon 18 of the human CFB gene, such as from the start codon to the stop codon, or the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof; further preferably including SEQ ID NO: 7 or 39). In some embodiments, a nucleotide sequence encoding a human CFB protein replaces all or a portion of the nucleotide sequence encoding the endogenous CFB protein.

In some embodiments, the humanized CFB gene comprises, sequentially from the 5' end to the 3' end: a portion of mouse CFB gene exon 1, a portion of human CFB gene exon 1 to a portion of exon 18, and a portion of mouse CFB gene exon 18. In some embodiments, the humanized CFB gene comprises, sequentially from the 5' end to the 3' end: a portion of mouse CFB gene exon 1, the 5'UTR of human CFB and at least a 50 bp contiguous nucleotide sequence upstream thereof, a nucleotide sequence from the start codon to the stop codon of the human CFB gene, the 3'UTR of human CFB and at least a 50 bp contiguous nucleotide sequence downstream thereof, a portion of mouse CFB gene intron 17, and mouse CFB gene exon 18. In some embodiments, in the humanized CFB gene, the connection sequence between the upstream of the nucleotide sequence of human CFB and the mouse sequence is SEQ ID NO: 9, and the connection sequence between the downstream of the nucleotide sequence of human CFB and the mouse sequence is SEQ ID NO: 10. In some embodiments, in the humanized CFB gene, the connection sequence between the upstream of the nucleotide sequence of human CFB and the mouse sequence is SEQ ID NO: 41, and the connection sequence between the downstream of the nucleotide sequence of human CFB and the mouse sequence is SEQ ID NO: 42. In some embodiments, the humanized CFB gene comprises, sequentially from the 5' end to the 3' end, one of the following: A) SEQ ID NO: 3, 7, and 4; B) SEQ ID NO: 3, 39, and 4; C) SEQ ID NO: 5, 7, and 6; D) SEQ ID NO: 5, 39, and 6; E) SEQ ID NO: 37, 7, and 38; F) SEQ ID NO: 37, 39, and 38; G) SEQ ID NO: 45, 7, and 46; H) SEQ ID NO: 45, 39, and 46. In some embodiments, the humanized CFB gene comprises one or more selected from the group consisting of SEQ ID NO: 11, 12, 43, and 44. In some embodiments, an mRNA transcribed from the humanized CFB gene comprises SEQ ID NO: 8 or 40, or comprises a nucleotide sequence that is at least 70%, at least 75%, at least 80%, at least 85%, at least 90%, at least 95%, or at least 99% identical to SEQ ID NO: 8 or 40, or comprises a nucleotide sequence that differs from SEQ ID NO: 8 or 40 by no more than 50, 40, 30, 20, 10, 9, 6, 3, or 1 nucleotide(s), or comprises a nucleotide sequence comprising a substitution, a deletion and/or insertion of one or more nucleotide(s) set forth in SEQ ID NO: 8 or 40. In some embodiments, the humanized CFB gene further comprises a resistance gene; preferably, the humanized CFB gene further comprises two Frt recombination sites arranged in the same orientation flanking the resistance gene. In some embodiments, the resistance gene is the neomycin phosphotransferase-encoding gene Neo. Preferably, the humanized CFB gene further comprises a specific inducer or repressor; further preferably, the specific inducer or repressor can be a conventionally inducible or repressible substance. In a specific embodiment of the disclosure, the specific inducer includes, but is not limited to, the tetracycline system (Tet-Off System/Tet-On System) or the tamoxifen system (Tamoxifen System). Preferably, the humanized CFB gene is regulated in the non-human animal by a regulatory element; further preferably, the regulatory element is an endogenous regulatory element or an exogenous regulatory element (e.g., a human regulatory element). Preferably, the regulatory element is a promoter.

In some embodiments, a portion of endogenous exon 1, intron 1, exon 2, intron 2, exon 3, intron 3, exon 4, intron 4, exon 5, intron 5, exon 6, intron 6, exon 7, intron 7, exon 8, intron 8, exon 9, intron 9, exon 10, intron 10, exon 11, intron 11, exon 12, intron 12, exon 13, intron 13, exon 14, intron 14, exon 15, intron 15, exon 16, intron 16, exon 17, intron 17, and/or a portion of exon 18 are deleted.

In some embodiments, a portion of endogenous exon 1, intron 1, exon 2, intron 2, exon 3, intron 3, exon 4, intron 4, exon 5, intron 5, exon 6, intron 6, exon 7, intron 7, exon 8, intron 8, exon 9, intron 9, exon 10, intron 10, exon 11, intron 11, exon 12, intron 12, exon 13, intron 13, exon 14, intron 14, exon 15, intron 15, exon 16, intron 16, exon 17, and a portion of intron 17 are deleted.

In some embodiments, the disclosure is related to a genetically modified non-human animal, wherein the genome of the non-human animal comprises a human, chimeric, or humanized CFB gene. In some embodiments, a protein encoded by the nucleotide sequence of the human, chimeric, or humanized CFB gene has at least 70%, at least 80%, at least 85%, at least 90%, at least 91%, at least 92%, at least 93%, at least 94%, at least 95%, at least 96%, at least 97%, at least 98%, at least 99%, or 100% identity to the amino acid sequence set forth in SEQ ID NO: 2 or amino acids 26-764 thereof. In some embodiments, a nucleotide sequence comprised in the genome of the non-human animal has at least 70%, at least 80%, at least 85%, at least 90%, at least 91%, at least 92%, at least 93%, at least 94%, at least 95%, at least 96%, at least 97%, at least 98%, at least 99%, or 100% identity to a nucleotide sequence set forth in SEQ ID NO: 3, 4, 5, 6, 7, 8, 9, 10, 11, 12, 37, 38, 39, 40, 41, 42, 43, 44, 45, or 46.

In some embodiments, the non-human animal of the present disclosure comprises a human or humanized CFB gene. In some embodiments, the humanized CFB gene comprises eighteen exons. In some embodiments, the humanized CFB gene comprises human or humanized exon 1, human exon 2, human exon 3, human exon 4, human exon 5, human exon 6, human exon 7, human exon 8, human exon 9, human exon 10, human exon 11, human exon 12, human exon 13, human exon 14, human exon 15, human exon 16, human exon 17, and/or human or humanized exon 18. In some embodiments, the humanized CFB gene comprises human intron 1, human intron 2, human intron 3, human intron 4, human intron 5, human intron 6, human intron 7, human intron 8, human intron 9, human intron 10, human intron 11, human intron 12, human intron 13, human intron 14, human intron 15, human intron 16, and/or human or humanized intron 17. In some embodiments, the humanized CFB gene comprises a human, endogenous, or humanized 5'UTR. In some embodiments, the humanized CFB gene comprises a human, endogenous, or humanized 3'UTR. In some embodiments, the humanized CFB gene comprises an endogenous or human 5'UTR. In some embodiments, the humanized CFB gene comprises an endogenous or human 3'UTR.

In some embodiments, the genetically modified non-human animal express human CFB and/or chimeric (e.g., humanized) CFB, wherein the endogenous CFB gene sequence is replaced by a human CFB gene and/or nucleotide sequence. Furthermore, the human CFB gene and/or nucleotide sequence encodes an amino acid sequence of human CFB that has at least 10%, at least 20%, at least 30%, at least 40%, at least 50%, at least 60%, at least 70%, at least 80%, at least 90%, at least 95%, at least 96%, at least 97%, at least 98%, or 100% identity to the amino acid sequence of human CFB set forth in SEQ ID NO: 2 or amino acids 26-764 thereof. In some embodiments, the nucleotide sequence of the endogenous CFB gene of the non-human animal is replaced in whole or in part by a nucleotide sequence encoding the mature human CFB protein.

In some embodiments, the genetically modified non-human animal expresses human CFB and/or chimeric CFB protein (e.g., humanized CFB protein) under the control of an endogenous or human-derived regulatory element (e.g., a promoter). Replacement at the endogenous gene locus provides a non-human animal that expresses the human or chimeric CFB protein (e.g., humanized CFB protein) in appropriate cell types. The genetically modified mice do not result in the potential pathologies observed in some other transgenic mice known in the art. The human CFB or chimeric CFB protein expressed in the non-human animal can maintain one or more functions of the wild-type or human CFB protein; for example, the expressed CFB protein can bind to human or non-human CFB protein. Furthermore, in some embodiments, the genetically modified non-human animal does not express endogenous CFB protein. In some embodiments, the genetically modified non-human animal expresses a decreased level of endogenous CFB protein. The term "endogenous CFB protein" as used herein refers to the CFB protein encoded by the endogenous CFB nucleotide sequence of the non-human animal (e.g., mouse) before genetic modification.

The genome of the non-human animal comprises a nucleotide sequence encoding an amino acid sequence that has at least 70%, at least 75%, at least 80%, at least 85%, at least 90%, at least 95%, at least 99%, or 100% identity to the amino acid sequence of human CFB protein (NP_001701.2; SEQ ID NO: 2). In some embodiments, the genome comprises a nucleotide sequence that has at least 70%, at least 75%, at least 80%, at least 85%, at least 90%, at least 95%, at least 99%, or 100% identity to the nucleotide sequence set forth in SEQ ID NO: 7, SEQ ID NO: 8, SEQ ID NO: 39, or SEQ ID NO: 40.

A nucleotide sequence encoding an endogenous CFB region in the genome of the non-human animal is replaced by a nucleotide sequence encoding human CFB. In some embodiments, the nucleotide sequence encoding the endogenous CFB region is any sequence at the endogenous CFB gene locus, such as exon 1, exon 2, exon 3, exon 4, exon 5, exon 6, exon 7, exon 8, exon 9, exon 10, exon 11, exon 12, exon 13, exon 14, exon 15, exon 16, exon 17, exon 18, 5'UTR, 3'UTR, intron 1, intron 2, intron 3, intron 4, intron 5, intron 6, intron 7, intron 8, intron 9, intron 10, intron 11, intron 12, intron 13, intron 14, intron 15, intron 16, intron 17, or any combination thereof. In some embodiments, the nucleotide sequence encoding the endogenous CFB region is located within an endogenous CFB regulatory region. In some embodiments, the nucleotide sequence encoding the endogenous CFB region is exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18, or a portion thereof.

The genetically modified non-human animal has one or more cells expressing human or chimeric CFB (e.g., a humanized CFB protein). In some embodiments, the human or chimeric CFB protein comprises at least 1, 2, 3, 4, 5, 6, 7, 8, 9, 10, 20, 30, 40, 50, 60, 70, 80, 90, 100, 150, 200, 220, 240, 260, 280, 300, 310, 350, 400, 450, 500, 550, 600, 650, 700, 710, 720, 730, 739, 740, 750, 760, 761, 762, 763, or 764 contiguous amino acids identical to the amino acid sequence set forth in SEQ ID NO: 2 or amino acids 26-764 thereof.

In some embodiments, the genome of the genetically modified non-human animal comprises all or a portion of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the human CFB gene, or all or a portion of the nucleotide sequence set forth in SEQ ID NO: 7, 8, 39, or 40.

In some embodiments, the genome of the genetically modified non-human animal comprises a portion of human CFB gene exon 1, all of exons 2-17, and a portion of exon 18, and preferably further comprises intron 1 and/or intron 17. In some embodiments, the portion of exon 1 comprises at least 5, 10, 20, 30, 40, 45, 50, 55, 60, 61, 62, 63, 64, 65, 70, 75, 80, 90, 100, 110, 120, 130, 140, 150, 160, 170, 180, 190, or 191 bp of contiguous nucleotide sequence from human CFB gene exon 1. In some embodiments, the portion of exon 1 comprises a 64 bp contiguous nucleotide sequence. In some embodiments, the portion of exon 1 comprises at least a 30 bp nucleotide sequence, preferably comprising a nucleotide sequence of the coding region within exon 1. In some embodiments, the portion of exon 18 comprises at least 20, 30, 40, 50, 60, 70, 80, 90, 100, 150, 151, 152, 153, 154, 155, 156, 157, 158, 159, 160, 170, 180, 190, 200, or 210 bp of contiguous nucleotide sequence from human CFB gene exon 18. In some embodiments, the portion of exon 18 comprises a 156 bp contiguous nucleotide sequence. In some embodiments, the portion of exon 18 comprises at least a 50 bp nucleotide sequence, preferably comprising a nucleotide sequence of the coding region within exon 18. The portion of human CFB gene exon 1, the entirety of exons 2-17, and the portion of exon 18 comprise at least a 100-500 bp, 500-1000 bp, 1000-2000 bp, or 2000-2500 bp contiguous nucleotide sequence. In some embodiments, the genome of the genetically modified non-human animal comprises the entirety of exons 1 to 18 of the human CFB gene, further comprises the 5'UTR and/or the 3'UTR of human CFB, and further comprises at least a 50 bp to at least 10000 bp (e.g., 50, 100, 500, 1000, 2000, 3000, 4000, 5000, 5500, 5600, 5700, 5800, 5900, 6000, 6100, 6200, 7000, 8000, 9000, or 10000 bp, preferably 3000-8000 bp, further preferably 5000-7000 bp) contiguous nucleotide sequence upstream of the 5'UTR of human CFB and/or at least a 50 bp to at least 5000 bp (e.g., 50, 100, 500, 600, 700, 800, 900, 910, 920, 930, 940, 950, 1000, 1100, 1200, 1300, 1400, 1500, 2000, 3000, 4000, or 5000 bp, preferably 500-3000 bp, further preferably 500-1500 bp) contiguous nucleotide sequence downstream of the 3'UTR of human CFB. In some embodiments, the nucleotide sequence of human CFB is the nucleotide sequence 1-2476 or 128-2422 of the human CFB gene transcript NM_001710.6.

In some embodiments, the modified CFB gene of the non-human animal is homozygous or heterozygous with respect to the replacement at the endogenous CFB gene locus.

In some embodiments, the humanized CFB genome comprises the 5'UTR of the human CFB gene. In some embodiments, the humanized CFB genome comprises an endogenous (e.g., mouse) 5'UTR. In some embodiments, the humanized CFB genome comprises the 3'UTR of the human CFB gene. In some embodiments, the humanized CFB genome comprises an endogenous (e.g., mouse) 3'UTR. In appropriate cases, it may be reasonable to presume that the mouse and human CFB genes appear to be similarly regulated based on the similarity of their sequences. As described in the disclosure, the humanized CFB mouse comprises a replacement at the endogenous mouse gene locus, which retains or does not retain the endogenous mouse regulatory elements but includes the humanized CFB coding sequence. Both genetically modified mice that are heterozygous or homozygous for humanized CFB are grossly normal

In another aspect, the disclosure also provides a genetically-modified, non-human animal whose genome comprise a disruption in the animal's endogenous CFB gene

In some embodiments, the disruption of the endogenous CFB gene comprises deletion of one or more exons or part of exons selected from the group consisting of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the endogenous CFB gene.

In some embodiments, the disruption of the endogenous CFB gene further comprises deletion of one or more introns or part of introns selected from the group consisting of intron 1, intron 2, intron 3, intron 4, intron 5, intron 6, intron 7, intron 8, intron 9, intron 10, intron 11, intron 12, intron 13, intron 14, intron 15, intron 16, and/or intron 17 of the endogenous CFB gene.

In some embodiments, the deletion comprises deletion of at least 1 bp to 6143 bp of the endogenous CFB gene. In some embodiments, the deletion comprises at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 250, 300, 350, 400, 450, 500, 550, 600, 650, 700, 750, 800, 850, 900, 950, 1000, 1300, 1350, 1400, 1450, 1500, 1600, 1800, 2000, 2292, 2293, 2300, 2400, 2500, 2600, 2700, 2750, 2766, 2767, 3000, 4000, 5000, 5500, 5686, 5687, 5688, 5689, 5690, 5700, 5800, 5900, 6000, 6100, or 6143 bp of contiguous nucleotide sequence, or more nucleotides.

In some embodiments, the deletion comprises deletion of at least 1 bp to 7011 bp of the endogenous CFB gene. In some embodiments, the deletion comprises at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 250, 300, 350, 400, 450, 500, 550, 600, 650, 700, 750, 800, 850, 900, 950, 1000, 1300, 1350, 1400, 1450, 1500, 1600, 1800, 2000, 2292, 2293, 2300, 2400, 2500, 2600, 2700, 2750, 2766, 2767, 3000, 4000, 5000, 5500, 5686, 5687, 5688, 5689, 5690, 5700, 5800, 5900, 6000, 6100, 6143, 6150, 6200, 6300, 6400, 6500, 6600, 6700, 6800, 6900, 7000, 7010, or 7011 bp of contiguous nucleotide sequence, or more nucleotides.

In some embodiments, the deletion of the endogenous CFB gene comprises at least 50 bp to at least 2767 bp, or at least 50 bp to 2449 bp, e.g., 50, 60, 70, 80, 90, 100, 150, 200, 250, 300, 400, 500, 600, 700, 800, 900, 1000, 1500, 2000, 2080, 2085, 2100, 2200, 2290, 2292, 2295, 2449, 2500, 2600, 2700, 2750, 2760, 2765, or 2767 bp of contiguous nucleotide sequence, or more nucleotides (e.g., deletion of at least a 30 bp contiguous nucleotide sequence of exon 1, the entirety of exons 2-17, and at least a 50 bp contiguous nucleotide sequence of exon 18; preferably further deletion of intron 1 and/or intron 17; or, for example, deletion of at least a 10 bp contiguous nucleotide sequence of exon 1, the entirety of exons 2-17, and a portion of intron 17) from exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18.

The disclosure is related to a humanized mouse CFB genomic DNA sequence, a vector that expresses an amino acid sequence of a humanized CFB protein; a cell comprising said vector and a tissue comprising said cell.

Therefore, in some embodiments, the disclosure provides a chimeric (e.g., humanized) CFB nucleotide sequence and/or amino acid sequence, wherein in some embodiments, the chimeric CFB nucleotide sequence and/or amino acid sequence has at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, or 100% identity to the mouse endogenous CFB mRNA (e.g., NM_008198.2), the mouse CFB amino acid sequence (e.g., NP_032224.2, SEQ ID NO: 1), or a portion thereof (e.g., a portion of exon 1 (comprising the coding region and optionally the 5'UTR), a portion of exon 18 (comprising the coding region and optionally the 3'UTR)). In some embodiments, the chimeric nucleotide sequence has at least 1%, 2%, 3%, 4%, 5%, 6%, 7%, 8%, 9%, 10%, 15%, 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99%, or 100% identity to the human CFB mRNA sequence (e.g., NM_001710.6), the human CFB amino acid sequence (e.g., NP_001701.2, SEQ ID NO: 2), or a portion thereof (e.g., a portion of exon 1, exons 2-17, or the 5'UTR and at least a 50 bp contiguous nucleotide sequence upstream thereof, a nucleotide sequence from the start codon to the stop codon of the human CFB gene, and the 3'UTR and at least a 50 bp contiguous nucleotide sequence downstream thereof).

In some embodiments, a nucleotide sequence encoding mouse CFB (SEQ ID NO: 1) or amino acids 25-763 thereof is replaced by a nucleotide sequence encoding human CFB (SEQ ID NO: 2) or amino acids 26-764 thereof.

In some embodiments, a nucleotide sequence encoding mouse CFB (SEQ ID NO: 1) or amino acids 18-712 thereof is replaced by a nucleotide sequence encoding human CFB (SEQ ID NO: 2) amino acids.

In some embodiments, the nucleic acids as described herein is operably linked to a regulatory element (preferably a promoter), e.g., a human CFB promoter or an endogenous mouse CFB promoter, an inducible promoter, an enhancer, and/or a mouse or human regulatory element.

In some embodiments, the chimeric nucleic acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 nucleotides, e.g., contiguous or non-contiguous nucleotides) that are different from part of or the entire mouse CFB nucleotide sequence (e.g., a portion of exon 1, exons 2-17, and a portion of exon 18 of the mouse CFB gene transcript NM_008198.2, or exons 1-18 of the mouse CFB gene transcript NM_008198.2).

In some embodiments, the chimeric nucleic acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 nucleotides, e.g., contiguous or non-contiguous nucleotides) that is the same as part of or the entire mouse CFB nucleotide sequence (e.g., a portion of exon 1 and the entirety or a portion of exon 18 of the mouse CFB gene transcript NM_008198.2).

In some embodiments, the chimeric nucleic acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 nucleotides, e.g., contiguous or non-contiguous nucleotides) that are different from part of or the entire human CFB nucleotide sequence (e.g., a portion of exon 1 and the entirety or a portion of exon 18 of the human CFB gene transcript NM_001710.6.).

In some embodiments, the chimeric nucleic acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 nucleotides, e.g., contiguous or non-contiguous nucleotides) that is the same as part of or the entire human CFB nucleotide sequence (e.g., a portion of exon 1, exons 2-17, and a portion of exon 18 of the human CFB gene transcript NM_001710.6; or, for example, exons 1-18 of the human CFB gene transcript NM_001710.6.).

In some embodiments, the amino acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 amino acid residues, e.g., contiguous or non-contiguous amino acid residues) that is different from part of or the entire mouse CFB amino acid sequence (e.g., the mouse CFB protein sequence NP_032224.2 (SEQ ID NO: 1) or amino acids 18-712 thereof.).

In some embodiments, the amino acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 amino acid residues, e.g., contiguous or non-contiguous amino acid residues) that is the same as part of or the entire mouse CFB amino acid sequence.

In some embodiments, the amino acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 amino acid residues, e.g., contiguous or non-contiguous amino acid residues) that is different from part of or the entire human CFB amino acid sequence.

In some embodiments, the amino acid sequence has at least a portion (e.g., at least 1, 2, 3, 4, 5, 6, 7, 8, 9, 10, 11, 12, 13, 14, 15, 20, 30, 40, 50, 60, 70, 80, 90, or 100 amino acid residues, e.g., contiguous or non-contiguous amino acid residues) that is the same as part of or the entire human CFB amino acid sequence (e.g., the human CFB protein sequence NP_001701.2 (SEQ ID NO: 2) or amino acids 26-764 thereof.).

The disclosure also provides a humanized CFB protein, the amino acid, wherein the amino acid sequence is selected from the group consisting of:
A) an amino acid sequence set forth in SEQ ID NO: 2 or amino acids 26-764 thereof;
B) an amino acid sequence having at least 90%, at least 91%, at least 92%, at least 93%, at least 94%, at least 95%, at least 96%, at least 97%, at least 98%, or at least 99% identity to the amino acid sequence set forth in SEQ ID NO: 2 or amino acids 26-764 thereof;
C) an amino acid sequence encoded by a nucleic acid sequence that can hybridize under low-stringency or stringent conditions to a nucleotide sequence encoding the amino acid sequence set forth in SEQ ID NO: 2 or amino acids 26-764 thereof;
D) an amino acid sequence that differs from the amino acid sequence set forth in SEQ ID NO: 2 or amino acids 26-764 thereof by no more than 10, 9, 8, 7, 6, 5, 4, 3, 2, or 1 amino acid(s); or
E) an amino acid sequence comprising a substitution, a deletion and/or insertion of one or more amino acid residue(s) in the amino acid sequence set forth in SEQ ID NO: 2 or amino acids 26-764 thereof.

The disclosure further provides a humanized CFB nucleic acid (e.g., DNA or RNA), wherein the nucleic acid sequence can be selected from the group consisting of:
A) a nucleotide sequence set forth in SEQ ID NO: 7, 8, 39, or 40, or a nucleic acid sequence encoding an amino acid sequence homologous to the humanized mouse CFB;
B) a nucleic acid sequence that can hybridize to the nucleotide sequence set forth in SEQ ID NO: 7, 8, 39, or 40 under low-stringency or stringent conditions;
C) a nucleic acid sequence that has a homology of at least 90%, 91%, 92%, 93%, 94%, 95%, 96%, 97%, 98%, or 99%, or at least 90%, 91%, 92%, 93%, 94%, 95%, 96%, 97%, 98%, or 99% identical to the nucleotide sequence set forth in SEQ ID NO: 7, 8, 39, or 40;
D) a nucleic acid sequence that encodes an amino acid sequence, wherein the amino acid sequence has at least 90%, at least 91%, at least 92%, at least 93%, at least 94%, at least 95%, at least 96%, at least 97%, at least 98%, at least 99%, or 100% identity to the amino acid sequence set forth in SEQ ID NO: 2 or amino acids 26-764 thereof;
E) a nucleic acid sequence that encodes an amino acid sequence, wherein the amino acid sequence is different from the amino acid sequence set forth in SEQ ID NO: 2 or amino acids 26-764 thereof by no more than 10, 9, 8, 7, 6, 5, 4, 3, 2, or 1 amino acid(s); or
F) a nucleic acid sequence that encodes an amino acid sequence, wherein the amino acid sequence comprises a substitution, a deletion and/or insertion of one or more amino acid residue(s) in the amino acid sequence set forth in SEQ ID NO: 2 or amino acids 26-764 thereof.

The present disclosure further relates to a CFB genomic DNA sequence of a humanized mouse. The DNA sequence is obtained by reverse transcription of the mRNA obtained by transcription thereof is consistent with or complementary to the DNA sequence homologous to the sequence shown in SEQ ID NO: 7, 8, 39 or 40.

To determine the percent identity of two amino acid sequences, or of two nucleic acid sequences, the sequences are aligned for optimal comparison purposes (e.g., gaps can be introduced in one or both of a first and a second amino acid or nucleic acid sequence for optimal alignment and non-homologous sequences can be disregarded for comparison purposes). The amino acid residues or nucleotides at corresponding amino acid positions or nucleotide positions are then compared. When a position in the first sequence is occupied by the same amino acid residue or nucleotide as the corresponding position in the second sequence, then the molecules are identical at that position. The percent identity between the two sequences is a function of the number of identical positions shared by the sequences, taking into account the number of gaps, and the length of each gap, which need to be introduced for optimal alignment of the two sequences. For example, the comparison of sequences and determination of percent identity between two sequences can be accomplished using a Blossum 62 scoring matrix with a gap penalty of 12, a gap extend penalty of 4, and a frameshift gap penalty of 5.

The percentage of residues conserved with similar physicochemical properties (percent homology), e.g. leucine and isoleucine, can also be used to measure sequence similarity. Families of amino acid residues having similar physicochemical properties have been defined in the art. These families include amino acids with basic side chains (e.g., lysine, arginine, histidine), acidic side chains (e.g., aspartic acid, glutamic acid), uncharged polar side chains (e.g., glycine, asparagine, glutamine, serine, threonine, tyrosine, cysteine), nonpolar side chains (e.g., alanine, valine, leucine, isoleucine, proline, phenylalanine, methionine, tryptophan), beta-branched side chains (e.g., threonine, valine, isoleucine) and aromatic side chains (e.g., tyrosine, phenylalanine, tryptophan, histidine). The homology percentage, in many cases, is higher than the identity percentage.

In some embodiments, the term "identity" refers to, in the context of using an amino acid sequence or a nucleotide sequence, modifications that can be made by a person skilled in the art based on practical needs while maintaining a similar structure or function to a known sequence. Compared to a sequence obtained from prior art, the sequence used may have (including but not limited to) 1%, 2%, 3%, 4%, 5%, 6%, 7%, 8%, 9%, 10%, 11%, 12%, 13%, 14%, 15%, 16%, 17%, 18%, 19%, 20%, 21%, 22%, 23%, 24%, 25%, 26%, 27%, 28%, 29%, 30%, 31%, 32%, 33%, 34%, 35%, 36%, 37%, 38%, 39%, 40%, 41%, 42%, 43%, 44%, 45%, 46%, 47%, 48%, 49%, 50%, 51%, 52%, 53%, 54%, 55%, 56%, 57%, 58%, 59%, 60%, 70%, 80%, 81%, 82%, 83%, 84%, 85%, 86%, 87%, 88%, 89%, 90%, 91%, 92%, 93%, 94%, 95%, 96%, 97%, 98%, 99%, 99.1%, 99.2%, 99.3%, 99.4%, 99.5%, 99.6%, 99.7%, 99.8%, or 99.9% identity or similarity.

The disclosure further provides cells, tissues, and animals (e.g., mice) comprising the nucleotide sequence described in the present application, as well as cells, tissues, and animals (e.g., mice) that express human or chimeric (e.g., humanized) CFB at an endogenous non-human CFB gene locus.

### Genetically modified animals

As used herein, the term "genetically-modified non-human animal" refers to a non-human animal having exogenous DNA in at least one chromosome of the animal's genome. In some embodiments, at least one or more cells, e.g., at least 1%, 2%, 3%, 4%, 5%, 10%, 20%, 30%, 40%, 50% of cells of the genetically-modified non-human animal have the exogenous DNA in its genome. The cell having exogenous DNA can be various kinds of cells, e.g., an endogenous cell, a somatic cell, an immune cell, a T cell, a B cell, an NK cell, an antigen presenting cell, a macrophage, a dendritic cell, a germ cell, a blastocyst, or an endogenous tumor cell. In some embodiments, genetically-modified non-human animals are provided that comprise a modified endogenous CFB locus that comprise an exogenous sequence (e.g., a human sequence), e.g., a replacement of one or more non-human sequences with one or more human sequences, or an insertion of one or more human and/or non-human sequences. The animals are generally able to pass the modification to progeny, i.e., through germline transmission.

As used herein, the term "chimeric (x) gene" or "chimeric (x) nucleic acid" refers to a gene or a nucleic acid, wherein two or more portions of the gene or the nucleic acid are from different species, or at least one of the sequences of the gene or the nucleic acid does not correspond to the wild-type nucleic acid in the animal. In some embodiments, the chimeric (x) gene or chimeric (x) nucleic acid has at least one portion of the sequence that is derived from two or more different sources, e.g., sequences encoding different proteins or sequences encoding the same (or homologous) protein of two or more different species. In some embodiments, the chimeric (x) gene or the chimeric (x) nucleic acid is a humanized gene or humanized nucleic acid.

As used herein, the term "chimeric (x) protein" or "chimeric (x) polypeptide" refers to a protein or a polypeptide, wherein two or more portions of the protein or the polypeptide are from different species, or at least one of the sequences of the protein or the polypeptide does not correspond to wild-type amino acid sequence in the animal. In some embodiments, the chimeric (x) protein or the chimeric (x) polypeptide has at least one portion of the sequence that is derived from two or more different sources, e.g., same (or homologous) proteins of different species. In some embodiments, the chimeric (x) protein or the chimeric (x) polypeptide is a humanized protein or a humanized polypeptide.

As used herein, the term "humanized (x) protein" or "humanized (x) polypeptide" refers to a protein or a polypeptide, wherein at least a portion of the protein or the polypeptide is from the human protein or human polypeptide. In some embodiments, the humanized protein or polypeptide is a human protein or polypeptide.

As used herein, the term "humanized (x) nucleic acid" or "humanized (x) gene" refers to a nucleic acid, wherein at least a portion of nucleic acid is from the human. In some embodiments, the entire nucleic acid in the humanized (x) nucleic acid or humanized (x) gene is from a human. In some embodiments, the humanized (x) nucleic acid or humanized (x) gene refers to a humanized exon, which can be a human exon or a chimeric exon. In some embodiments, the humanized (x) nucleic acid or humanized (x) gene refers to a humanized exon and a humanized intron, wherein the humanized intron can be a human intron or a chimeric intron.

In some embodiments, the chimeric (x) gene or the chimeric (x) nucleic acid is a humanized CFB gene or a humanized CFB nucleic acid. In some embodiments, at least one or more portions of the gene or the nucleic acid is from the human CFB gene, at least one or more portions of the gene or the nucleic acid is from a non-human CFB gene. In some embodiments, the gene or the nucleic acid comprises a sequence that encodes an CFB protein. The encoded CFB protein is functional or has at least one activity of the human CFB protein or the non-human CFB protein.

In some embodiments, the chimeric (x) protein or the chimeric (x) polypeptide is a humanized CFB protein or a humanized CFB polypeptide. In some embodiments, at least one or more portions of the amino acid sequence of the protein or the polypeptide is from a human CFB protein, and at least one or more portions of the amino acid sequence of the protein or the polypeptide is from a non-human CFB protein. The humanized CFB protein or the humanized CFB polypeptide is functional or has at least one activity of the human CFB protein or the non-human CFB protein.

The genetically modified non-human animal can be various animals, e.g., a mouse, rat, zebrafish, rabbit, pig, bovine (e.g., cow, bull, buffalo), deer, sheep, goat, chicken, cat, dog, ferret, primate (e.g., marmoset, rhesus monkey). For the non-human animals where suitable genetically modifiable embryonic stem (ES) cells are not readily available, other methods are employed to make a non-human animal comprising the genetic modification. Such methods include, e.g., modifying a non-ES cell genome (e.g., a fibroblast or an induced pluripotent cell) and employing nuclear transfer to transfer the modified genome to a suitable cell, e.g., an oocyte, and gestating the modified cell (e.g., the modified oocyte) in a non-human animal under suitable conditions to form an embryo. These methods are known in the art, and are described, e.g., in A. Nagy, et al., "Manipulating the Mouse Embryo: A Laboratory Manual (Third Edition)," Cold Spring Harbor Laboratory Press, 2003, which is incorporated by reference herein in its entirety.

In one aspect, the animal is a mammal. In some embodiments, the genetically modified animal is a rodent. The rodent can be selected from a mouse, a rat, and a hamster. In some embodiments, the genetically modified animal is from a family selected from *Calomyscidae* (e.g., mouse-like hamsters), *Cricetidae* (e.g., hamster, New World rats and mice, voles), *Muridae* (true mice and rats, gerbils, spiny mice, crested rats), *Nesomyidae* (climbing mice, rock mice, with-tailed rats, Malagasy rats and mice), *Platacanthomyidae* (e.g., spiny dormice), and *Spalacidae* (e.g., mole rates, bamboo rats, and zokors). In some embodiments, the genetically modified rodent is selected from a true mouse or rat (family *Muridae*), a gerbil, a spiny mouse, and a crested rat. In one embodiment, the genetically modified mouse is derived from a member of the *Muridae* family. In one embodiment, the non-human animal is a rodent. In a specific embodiment, the rodent is selected from a mouse and a rat. In some embodiments, the non-human animal is a mouse.

In some embodiments, the non-human animal can be an immunodeficient non-human mammal. Examples include an immunodeficient rodent, an immunodeficient rabbit, an immunodeficient pig, an immunodeficient monkey, and the like.

In some embodiments, the animal is a mouse of a C57BL strain selected from C57BL/a, C57BL/An, C57BL/GrFa, C57BL/KaLwN, C57BL/min, C57BL/6J, C57BL/6ByJ, C57BL/6NJ, C57BL/10, C57BL/10ScSn, C57BL/10Cr, and C57BL/Ola. In some embodiments, the mouse is a 129 strain selected from the group consisting of a strain that is 129P1, 129P2, 129P3, 129X1, 129S1 (e.g., 129S1/SV, 129S1/SvIm), 129S2, 129S4, 129S5, 129S9/SvEvH, 129S6 (129/SvEvTac), 129S7, 129S8, 129T1, 129T2. These mice are described, e.g., in Festing et al., Revised nomenclature for strain 129 mice, Mammalian Genome 10: 836 (1999); Auerbach et al., Establishment and Chimera Analysis of 129/SvEv- and C57BL/6-Derived Mouse Embryonic Stem Cell Lines (2000), both of which are incorporated herein by reference in the entirety. In some embodiments, the genetically modified mouse is a mix of the 129 strain and the C57BL/6 strain. In some embodiments, the mouse is a mix of the 129 strains, or a mix of the BL/6 strains. In some embodiments, the mouse is a BALB strain, e.g., BALB/c strain. In some embodiments, the mouse is a mix of a BALB strain and another strain. In some embodiments, the mouse is from a hybrid line (e.g., 50% BALB/c-50% 12954/Sv; or 50% C57BL/6-50% 129). In some embodiments, the non-human animal is a rodent. In some embodiments, the non-human animal is a mouse having a BALB/c, A, A/He, A/J, A/WySN, AKR, AKR/A, AKR/J, AKR/N, TA1, TA2, RF, SWR, C3H, C57BR, SJL, C57L, DBA/2, KM, NIH, ICR, CFW, FACA, C57BL/A, C57BL/An, C57BL/GrFa, C57BL/KaLwN, C57BL/6, C57BL/6J, C57BL/6ByJ, C57BL/6NJ, C57BL/10, C57BL/10ScSn, C57BL (C57BL/10Cr and C57BL/Ola), C58, CBA/Br, CBA/Ca, CBA/J, CBA/st, or CBA/H background, and mice with backgrounds such as NOD, NOD/SCID, and NOD-Prkdc^{scid} IL-2rg^{null}.

Genetically modified non-human animals can comprise a modification at endogenous non-human CFB locus. In some embodiments, the modification can comprise a human nucleic acid sequence encoding at least a portion of a mature CFB protein (e.g., at least 10%, 20%, 30%, 40%, 50%, 60%, 70%, 80%, 90%, 95%, 96%, 97%, 98%, or 99% identical to the mature CFB protein sequence). Although genetically modified cells are also provided that can comprise the modifications described herein (e.g., ES cells, somatic cells), in many embodiments, the genetically modified non-human animals comprise the modification of the endogenous CFB locus in the germline of the animal.

In some embodiments, the genetically modified non-human animal (e.g., a mouse) can express human CFB and/or chimeric CFB (e.g., humanized CFB) under the control of non-human animal endogenous or human-derived regulatory elements (including 5'UTR, 3'UTR, enhancers, or promoters). Insertion or replacement at the endogenous gene locus of the non-human animal (e.g., mouse) provides a non-human animal that expresses human CFB or chimeric CFB (e.g., humanized CFB) in appropriate cell types and does not result in the potential pathologies observed in some other transgenic mice known in the art. The human CFB or chimeric CFB (e.g., humanized CFB) expressed in the non-human animal can maintain one or more functions of wild-type mouse or human CFB in the non-human animal. Furthermore, in some embodiments, the non-human animal does not express endogenous CFB. In some embodiments, the expression level of endogenous CFB in the non-human animal is decreased as compared to the CFB expression level in a wild-type animal. The term "endogenous CFB" as used in the disclosure refers to the CFB protein expressed by the endogenous CFB nucleotide sequence of the non-human animal (e.g., mouse) before any genetic modification.

The genome of the genetically modified non-human animal comprises a nucleotide sequence encoding an amino acid sequence having at least 70%, at least 75%, at least 80%, at least 85%, at least 90%, at least 95%, at least 99%, or 100% identity to human CFB (NP_001701.2; SEQ ID NO: 2). In some embodiments, the genome comprises a nucleotide sequence having at least 70%, at least 75%, at least 80%, at least 85%, at least 90%, at least 95%, at least 99%, or 100% identity to SEQ ID NO: 7, 8, 39, or 40. In some embodiments, the genome comprises a nucleotide sequence having at least 70%, at least 75%, at least 80%, at least 85%, at least 90%, at least 95%, at least 99%, or 100% identity to 1-2476 or 128-2422 of NM_001710.6.

In some embodiments, the genome of the non-human animal comprises: a portion of exon 1, exon 2, exon 3, exon 4, exon 5, exon 6, exon 7, exon 8, exon 9, exon 10, exon 11, exon 12, exon 13, exon 14, exon 15, exon 16, exon 17, and/or a portion of exon 18 of the human CFB gene; or, the entirety of exons 1-18 of the human CFB gene; or, the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof; or a nucleotide sequence encoding all or a portion of the amino acid sequence of human CFB; or a nucleotide sequence encoding SEQ ID NO: 2 or amino acids 26-764 thereof.

In some embodiments, the genome of the genetically modified non-human animal comprises a portion of exon 1, the entirety of exons 2-17, and a portion of exon 18 of the human CFB gene, and preferably further comprises intron 1 and/or intron 17. In some embodiments, the portion of exon 1 comprises at least a 5 bp to 191 bp contiguous nucleotide sequence, e.g., 5, 10, 20, 30, 40, 45, 50, 55, 60, 61, 62, 63, 64, 65, 70, 75, 80, 90, 100, 110, 120, 130, 140, 150, 160, 170, 180, 190, or 191 nucleotides. In some embodiments, the portion of exon 1 comprises 64 nucleotides. In some embodiments, the portion of exon 1 comprises at least 30 nucleotides. In some embodiments, the portion of exon 18 comprises at least a 20 bp to 210 bp contiguous nucleotide sequence, e.g., 20, 30, 40, 50, 60, 70, 80, 90, 100, 150, 151, 152, 153, 154, 155, 156, 157, 158, 159, 160, 170, 180, 190, 200, or 210 nucleotides. In some embodiments, the portion of exon 18 comprises 156 nucleotides. In some embodiments, the portion of exon 18 comprises at least 50 nucleotides.

In some embodiments, the genome of the genetically modified non-human animal comprises a portion of exon 1 and exon 18 of the endogenous CFB gene (e.g., mouse CFB). In some embodiments, the portion of exon 1 comprises at least 1, 2, 3, 4, 5, 6, 10, 20, 50, 100, 150, 151, 152, 153, 154, 155, 156, 157, 158, 159, 160, 170, 180, 190, 200, 300, 400, 401, 402, 403, 404, 405, 410, 450, 460, or 463 nucleotides. In some embodiments, the portion of exon 1 comprises 452 nucleotides. In some embodiments, the portion of exon 1 comprises at least 100 bp, 150 bp, or 200 bp of nucleotides.

In some embodiments, the genome of the genetically modified non-human animal comprises a portion of exon 1 and a portion of exon 18 of the endogenous CFB gene (e.g., mouse CFB). In some embodiments, the portion of exon 1 comprises at least 1, 2, 3, 4, 5, 6, 10, 20, 50, 100, 150, 151, 152, 153, 154, 155, 156, 157, 158, 159, 160, 170, 180, 190, 200, 300, 400, 401, 402, 403, 404, 405, 410, 450, 460, or 463 nucleotides. In some embodiments, the portion of exon 1 comprises 402 or 155 nucleotides. In some embodiments, the portion of exon 1 comprises at least 100 bp, 150 bp, or 200 bp of nucleotides. In some embodiments, the portion of exon 18 comprises at least 5, 6, 7, 8, 9, 10, 20, 30, 40, 45, 50, 51, 52, 53, 54, 55, 60, 70, 80, 90, 100, 150, 200, 205, 208, or 209 nucleotides. In some embodiments, the portion of exon 18 comprises 209 or 53 nucleotides. In some embodiments, the portion of exon 18 comprises at least 20 bp or 150 bp of nucleotides.

In some embodiments, the non-human animal has, at the endogenous CFB gene locus, a nucleotide sequence encoding a chimeric human/non-human CFB polypeptide, and the non-human animal expresses functional CFB on its cell surface. The human portion of the chimeric human/non-human CFB polypeptide may comprise an amino acid sequence encoded by a portion of exon 1, exons 2-17, and/or a portion of exon 18 of the human CFB gene, or an amino acid sequence encoded by the entirety of exons 1-18 of the human CFB gene. In some embodiments, the human portion of the chimeric human/non-human CFB polypeptide comprises a sequence that is at least 80%, at least 85%, at least 90%, at least 95%, or at least 99% identical to SEQ ID NO: 2 or amino acids 26-764 thereof.

Furthermore, the modified gene in the genome of the modified non-human animal is homozygous or heterozygous with respect to the endogenous replaced gene locus. In a specific embodiment, the modified CFB gene in the genome is homozygous or heterozygous with respect to the endogenous replaced gene locus.

In some embodiments, the humanized CFB gene locus comprises a human 5'UTR. In some embodiments, the humanized CFB gene locus comprises a non-human animal endogenous (e.g., mouse) 5'UTR. In some embodiments, the humanized CFB gene locus comprises a human 3'UTR. In some embodiments, the humanized CFB gene locus comprises a non-human animal endogenous (e.g., mouse) 3'UTR. In appropriate cases, it may be reasonable to presume that the mouse and human CFB genes appear to be similarly regulated based on the similarity of their sequences. As shown in the application, a humanized CFB mouse comprising an insertion or replacement at the endogenous CFB gene locus of a non-human animal, which retains mouse regulatory elements but contains a humanized CFB coding sequence, does not exhibit pathological phenomena. Both heterozygous and homozygous genetically modified mice with the humanized CFB gene are normal.

In another aspect, the disclosure further provides a genetically modified non-human animal, the genome of which comprises a disruption of the endogenous CFB gene of the non-human animal, wherein the disruption of the endogenous CFB gene includes deletion of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18, or a portion thereof.

In some embodiments, the disruption of the endogenous CFB gene of the non-human animal further comprises deletion of one or more introns selected from intron 1, intron 2, intron 3, intron 4, intron 5, intron 6, intron 7, intron 8, intron 9, intron 10, intron 11, intron 12, intron 13, intron 14, intron 15, intron 16, and intron 17.

In some embodiments, the deletion may comprise deletion of at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 300, 400, 450, 460, 465, 466, 467, 468, 469, 470, 500, 600, 700, 800, 900, 1000, 2000, 3000, 4000, 5000, 5500, 5600, 5686, or more nucleotides.

In some embodiments, the disruption of the endogenous CFB gene includes deletion of at least 1, 2, 3, 4, 5, 6, 7, 8, 9, 10, 20, 30, 40, 50, 60, 70, 80, 90, 100, 110, 120, 130, 140, 150, 160, 170, 180, 190, 200, 210, 220, 230, 240, 250, 260, 270, 280, 290, or 300 nucleotides from exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18.

The disclosure further relates to a humanized mouse CFB genomic DNA sequence, a DNA sequence obtained by reverse transcription of mRNA that is identical or complementary to said DNA sequence; a construct expressing the amino acid sequence thereof; a cell comprising said construct; and a tissue comprising said cell.

The disclosure further relates to a non-human mammal generated by the aforementioned method. In some embodiments, the genome thereof contains human gene(s).

In some embodiments, the non-human mammal is a rodent, and preferably, the non-human mammal is a mouse.

In some embodiments, the non-human mammal expresses a protein encoded by humanized CFB gene.

In addition, the present disclosure also relates to a tumor bearing non-human mammal model, characterized in that the non-human mammal model is obtained through the methods as described herein. In some embodiments, the non-human mammal is a rodent (e.g., a mouse).

The present disclosure further relates to a cell or cell line, or a primary cell culture thereof derived from the non-human mammal or an offspring thereof, or the tumor bearing non-human mammal; the tissue, organ or a culture thereof derived from the non-human mammal or an offspring thereof, or the tumor bearing non-human mammal; and the tumor tissue derived from the non-human mammal or an offspring thereof when it bears a tumor, or the tumor bearing non-human mammal.

The present disclosure also provides non-human mammals produced by any of the methods described herein. In some embodiments, a non-human mammal, a genetically modified non-human animal is provided, wherein the genome of the genetically modified non-human animal comprises human or humanized CFB DNA.

In some embodiments, the non-human mammal comprises a gene construct as described in the present application (e.g., the gene constructs shown in **FIGs. 2-5** and **FIGs. 17-19**). In some embodiments, a non-human mammal expressing a human or humanized CFB protein is provided. In some embodiments, a tissue that specifically expresses a human or humanized CFB protein is provided.

In some embodiments, the expression of human or humanized CFB in a genetically modified animal is controllable, as by the addition of a specific inducer or repressor substance. In some embodiments, the specific inducer is selected from Tet-Off System/Tet-On System, or Tamoxifen System.

Non-human mammals can be any non-human animal known in the art and which can be used in the methods as described herein. Preferred non-human mammals are mammals, (e.g., rodents). In some embodiments, the non-human mammal is a mouse.

Genetic, molecular and behavioral analyses for the non-human mammals described above can performed. The present disclosure also relates to the progeny produced by the non-human mammal provided by the present disclosure mated with the same or other genotypes.

The present disclosure also provides a cell line or primary cell culture derived from the non-human mammal or a progeny thereof. A model based on cell culture can be prepared, for example, by the following methods. Cell cultures can be obtained by way of isolation from a non-human mammal, alternatively cells can be obtained from the cell culture established using the same constructs and the standard cell transfection techniques. The integration of genetic constructs containing DNA sequences encoding human CFB proteins can be detected by a variety of methods.

There are many analytical methods that can be used to detect exogenous DNA, including methods at the level of nucleic acid (including the mRNA quantification approaches using reverse transcriptase polymerase chain reaction (RT-PCR) or Southern blotting, and *in situ* hybridization) and methods at the protein level (including histochemistry, immunoblot analysis and *in vitro* binding studies). In addition, the expression level of the gene of interest can be quantified by ELISA techniques well known to those skilled in the art. Many standard analysis methods can be used to complete quantitative measurements. For example, transcription levels can be measured using RT-PCR and hybridization methods including RNase protection, Southern blot analysis, RNA dot analysis (RNAdot) analysis. Immunohistochemical staining, flow cytometry, Western blot analysis can also be used to assess the presence of human or humanized CFB proteins.

In some embodiments, the genetically modified non-human animal described in the present application (e.g., a CFB gene humanized homozygous mouse) can express human or humanized CFB in one or more liver tissue cells.

### Vectors

The present disclosure provides a targeting vector, comprising: a) a DNA fragment homologous to the 5' end of the region to be altered (5' arm), selected from the genomic DNA of the CFB gene with a length of 100 to 10,000 nucleotides; b) a donor DNA sequence encoding a donor region; and c) a second DNA fragment homologous to the 3' end of the region to be altered (3' arm), selected from the genomic DNA of the CFB gene with a length of 100 to 10,000 nucleotides.

In some embodiments, a) the DNA fragment homologous to the 5' end of the region to be altered is selected from a nucleotide sequence has at least 90% homology to NCBI accession number NC_000083.7; c) the DNA fragment homologous to the 3' end of the region to be altered is selected from a nucleotide sequence has at least 90% homology to NCBI accession number NC_000083.7.

In some embodiments, a) the DNA fragment homologous to the 5' end of the region to be altered is selected from the nucleotide sequence at position 35081089 to 35084696 of NCBI accession number NC_000083.7; c) the DNA fragment homologous to the 3' end of the region to be altered is selected from the nucleotide sequence at position 35070668 to 35074536 of NCBI accession number NC_000083.7.

In some embodiments, a) the DNA fragment homologous to the 5' end of the region to be altered is selected from the nucleotide sequence at position 35081089 to 35082539 of NCBI accession number NC_000083.7; c) the DNA fragment homologous to the 3' end of the region to be altered is selected from the nucleotide sequence at position 35073826 to 35075402 of NCBI accession number NC_000083.7.

In some embodiments, a) the DNA fragment homologous to the 5' end of the region to be altered is selected from the nucleotide sequence at position 35081039 to 35084904 of NCBI accession number NC_000083.7; c) the DNA fragment homologous to the 3' end of the region to be altered is selected from the nucleotide sequence at position 35071591 to 35075667 of NCBI accession number NC_000083.7.

In some embodiments, a) the DNA fragment homologous to the 5' end of the region to be altered is selected from the nucleotide sequence at position 35081039 to 35082302 of NCBI accession number NC_000083.7; c) the DNA fragment homologous to the 3' end of the region to be altered is selected from the nucleotide sequence at position 35074350 to 35075667 of NCBI accession number NC_000083.7.

In some embodiments, the length of the selected genomic nucleotide sequence in the targeting vector can be more than 3 kb, 3.5 kb, 4 kb, 4.5 kb, 5 kb, 5.5 kb, 5.8 kb, or 6 kb.

In some embodiments, the region to be altered is located at the endogenous CFB gene locus of the non-human animal. For example, it is located at exons 1 to 18 of the non-human animal CFB gene, or exons 1 to 17 of the non-human animal CFB gene.

In some embodiments, the targeting vector further comprises one or more marker genes. For example, a positive selection marker gene or a negative selection marker gene. In some embodiments, the positive selectable marker is a Neo gene or Neo cassette. Preferably, the targeting vector further comprises two recombination system Frt recombination sites arranged in the same direction located on both sides of the marker gene. In some embodiments, the negative selectable marker is a DTA gene.

In some embodiments, the 5' arm sequence is the nucleotide sequence as shown in SEQ ID NO: 3; the 3' arm sequence is the nucleotide sequence as shown in SEQ ID NO: 4. In some embodiments, the 5' arm sequence is the nucleotide sequence as shown in SEQ ID NO: 5; the 3' arm sequence is the nucleotide sequence as shown in SEQ ID NO: 6.

In some embodiments, the 5' arm sequence is the nucleotide sequence as shown in SEQ ID NO: 37; the 3' arm sequence is the nucleotide sequence as shown in SEQ ID NO: 38. In some embodiments, the 5' arm sequence is the nucleotide sequence as shown in SEQ ID NO: 45; the 3' arm sequence is the nucleotide sequence as shown in SEQ ID NO: 46.

In some embodiments, the 5' arm is a nucleotide that is at least 90% identical to NCBI accession number NC_000083.7. Further preferably, the 5' arm sequence comprises the nucleotide sequence shown in SEQ ID NO: 3 or 5. Further preferably, the 5' arm sequence comprises the nucleotide sequence shown in SEQ ID NO: 37 or 45. In some embodiments, the 3' arm is a nucleotide that is at least 90% identical to NCBI accession number NC_000083.7. Further preferably, the 3' arm sequence comprises the nucleotide sequence shown in SEQ ID NO: 4 or 6. Further preferably, the 3' arm sequence comprises the nucleotide sequence shown in SEQ ID NO: 38 or 46.

In some embodiments, the targeting vector comprises a human sequence (e.g., nucleotides 31946222 to 31952030 of NC_000006.12). For example, the donor region in the targeting vector comprises: a portion or all of the nucleotide sequence of the human CFB gene, preferably exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the human CFB gene. In some embodiments, the nucleotide sequence of the humanized CFB gene encodes all or a portion of the human CFB protein, the NCBI protein accession number of which is NP_001701.2 (SEQ ID NO: 2).

In some embodiments, the targeting vector comprises, from 5' end to 3' end, a portion of mouse CFB gene exon 1, a portion of human CFB gene exon 1 to a portion of exon 18, and a portion of mouse CFB gene exon 18.

In some embodiments, the targeting vector comprises, from 5' end to 3' end, a portion of mouse CFB gene exon 1, human CFB gene exon 1 to exon 18, and mouse CFB gene exon 18.

In some embodiments, the targeting vector comprises, from 5' end to 3' end, a portion of mouse CFB gene exon 1, the human CFB gene 5'UTR and at least 50 bp contiguous nucleotides upstream thereof, the nucleotide sequence from the start codon to the stop codon of the human CFB gene, the human CFB 3'UTR and at least 50 bp contiguous nucleotides downstream thereof, and mouse CFB gene exon 18.

In some embodiments, the connection sequence between the 5' end of the donor region sequence and the 5' homologous arm in the targeting vector is SEQ ID NO: 9, and the connection sequence between the 3' end of the donor region sequence and the 3' homologous arm is SEQ ID NO: 10.

In some embodiments, the connection sequence between the 5' end of the donor region sequence and the 5' homologous arm in the targeting vector is SEQ ID NO: 41, and the connection sequence between the 3' end of the donor region sequence and the 3' homologous arm is SEQ ID NO: 42.

In some embodiments, the targeting vector comprises, from 5' end to 3' end:
A) SEQ ID NO: 3, SEQ ID NO: 7, and SEQ ID NO: 4;
B) SEQ ID NO: 3, SEQ ID NO: 39, and SEQ ID NO: 4;
C) SEQ ID NO: 5, SEQ ID NO: 7, and SEQ ID NO: 6;
D) SEQ ID NO: 5, SEQ ID NO: 39, and SEQ ID NO: 6;
E) SEQ ID NO: 37, SEQ ID NO: 7, and SEQ ID NO: 38;
F) SEQ ID NO: 37, SEQ ID NO: 39, and SEQ ID NO: 38;
G) SEQ ID NO: 45, SEQ ID NO: 7, and SEQ ID NO: 46;
H) SEQ ID NO: 45, SEQ ID NO: 39, and SEQ ID NO: 46.

In some embodiments, the targeting vector comprises a Neo cassette. The Neo cassette can be inserted into the 5' homologous arm, the 3' homologous arm, or the donor region sequence.

In some embodiments, the targeting vector further comprises one or more selected from the group consisting of SEQ ID NO: 11, SEQ ID NO: 12, SEQ ID NO: 43, and SEQ ID NO: 44.

The present disclosure further provides sgRNA vectors for constructing humanized animal models or knockout models. In some embodiments, the vector comprises a sgRNA sequence, wherein the sgRNA sequence targets the CFB gene, and the sgRNA is unique to the target sequence of the gene to be altered and satisfies the sequence arrangement rule of 5'-NNN(20)-NGG3' or 5'-CCN-N(20)-3'; and in some embodiments, the targeting site of the sgRNA in the mouse CFB gene is located at exon 1, intron 1, exon 2, intron 2, exon 3, intron 3, exon 4, intron 4, exon 5, intron 5, exon 6, intron 6, exon 7, intron 7, exon 8, intron 8, exon 9, intron 9, exon 10, intron 10, exon 11, intron 11, exon 12, intron 12, exon 13, intron 13, exon 14, intron 14, exon 15, intron 15, exon 16, intron 16, exon 17, intron 17, exon 18, e.g., mouse CFB gene exon 1, intron 17 and/or exon 18.

In some embodiments, the targeting sequence is one or more shown as SEQ ID NO: 13, 14, 15, 16, 17, 18, 19, 20, 21, and 22. In some embodiments, the targeting sequence is one or more shown as SEQ ID NO: 47, 48, 49, 50, 51, 52, 53, 54, 55, and 56. Therefore, the present disclosure provides sgRNA sequences for making a genetically modified animal model. In some embodiments, the oligonucleotide sgRNA sequences are shown in SEQ ID NO: 15 and SEQ ID NO: 17. In some embodiments, the oligonucleotide sgRNA sequences are shown in SEQ ID NO: 16 and SEQ ID NO: 18. In some embodiments, the oligonucleotide sgRNA sequences are shown in SEQ ID NO: 19 and SEQ ID NO: 21. In some embodiments, the oligonucleotide sgRNA sequences are shown in SEQ ID NO: 20 and SEQ ID NO: 22. In some embodiments, the oligonucleotide sgRNA sequences are shown in SEQ ID NO: 49 and SEQ ID NO: 51. In some embodiments, the oligonucleotide sgRNA sequences are shown in SEQ ID NO: 50 and SEQ ID NO: 52. In some embodiments, the oligonucleotide sgRNA sequences are shown in SEQ ID NO: 53 and SEQ ID NO: 55. In some embodiments, the oligonucleotide sgRNA sequences are shown in SEQ ID NO: 54 and SEQ ID NO: 56.

In some embodiments, the present disclosure relates to a plasmid construct (e.g., pT7-sgRNA) comprising the sgRNA sequence and/or a cell comprising the construct.

The present disclosure further relates to a cell comprising the targeting vector as described above.

Furthermore, the present disclosure further provides a non-human mammalian cell comprising any one of the targeting vectors described above and one or more *in vitro* transcripts of the constructs described herein. In some embodiments, the cell comprises Cas9 mRNA or *in vitro* transcript thereof.

In some embodiments, the gene in the cell is heterozygous. In some embodiments, the gene in the cell is homozygous.

In some embodiments, the non-human mammalian cell is a mouse cell. In some embodiments, the cell is a fertilized egg cell. In some embodiments, the cell is an embryonic stem cell.

The present disclosure further relates to the use of the aforementioned targeting vector, sgRNA, or sgRNA vector in CFB gene modification.

### Methods of making genetically modified animals

Genetically modified animals can be made by several techniques that are known in the art, including, homologous recombination (HR) utilizing embryonic stem cells (including gene targeting technology, CRISPR/Cas9 gene editing technology), zinc finger nucleases (ZFNs), transcription activator-like effector nucleases (TALEN), homing endonucleases, or other molecular biology techniques. In some embodiments, homologous recombination is used. In some embodiments, CRISPR-Cas9 genome editing is used to generate genetically modified animals. Many of these genome editing techniques are known in the art, and is described, e.g., in Yin et al., "Delivery technologies for genome editing" Nature Reviews Drug Discovery 16.6 (2017): 387-399, which is incorporated by reference in its entirety. Many other methods are also provided and can be used in genome editing, e.g., micro-injecting a genetically modified nucleus into an enucleated oocyte, and fusing an enucleated oocyte with another genetically modified cell.

In some embodiments, the nucleotide sequence encoding the endogenous CFB region in the endogenous genome of at least one cell of a non-human animal is replaced with a nucleotide sequence encoding the corresponding region of human CFB. In some embodiments, the endogenous CFB protein of the non-human animal is expressed at a lower level or is not expressed compared to wild-type CFB. In some embodiments, the replacement occurs in a germ cell, a somatic cell, a blastocyst, or a fibroblast, etc. The nucleus of a somatic cell or the fibroblast can be inserted into an enucleated oocyte.

**FIG. 3****,** **FIG. 5****,** **FIG. 18,** and **FIG. 19** show a humanized targeting strategy for targeting the mouse CFB gene locus. The targeting vector comprises a vector composed of a 5' homology arm, a human or humanized CFB gene fragment, and a 3' homology arm. The process involves replacing the endogenous corresponding CFB nucleotide sequence with a human or humanized nucleotide sequence using homologous recombination. In some embodiments, cleavage upstream and downstream of the target site (e.g., by zinc finger nucleases, TALENs, or CRISPR) can result in double-strand DNA breaks, enabling the replacement of the mouse endogenous CFB sequence with a human or humanized CFB sequence through homologous recombination.

Therefore, in some embodiments, the method for generating a genetically modified humanized animal comprises replacing a nucleic acid sequence encoding the endogenous CFB region with a nucleotide sequence encoding the human CFB at the endogenous CFB gene locus. The sequence for replacing can comprise regions (e.g., portion or all of the regions) of exon 1, exon 2, exon 3, exon 4, exon 5, exon 6, exon 7, exon 8, exon 9, exon 10, exon 11, exon 12, exon 13, exon 14, exon 15, exon 16, exon 17, and/or exon 18 of the human CFB gene. In some embodiments, the sequence comprises portion of exon 1, exons 2-17, and portion of exon 18 of the human CFB gene (e.g., the nucleotide sequence at positions 1-2476 or 128-2422 of NM_001710.6). In some embodiments, the sequence comprises exon 1 to exon 18 of the human CFB gene (e.g., the nucleotide sequence at positions 1-2476 of NM_001710.6). In some embodiments, the sequence comprises portion of exon 1 and all or portion of exon 18 of the endogenous CFB gene (e.g., the nucleotide sequence at positions 1-402 and 2695-2747 of NM_008198.2, or the nucleotide sequence at positions 1-452 and 2539-2747 of NM_008198.2, or the nucleotide sequence at positions 1-155 and 2241-2449 of NM_008198.3).

The present disclosure further provides a method for establishing an IL5 gene humanized animal model, involving the following steps:
(a) providing the cell (e.g. a fertilized egg cell) based on the methods described herein;
(b) culturing the cell (preferably culturing the cell in a liquid culture medium);
(c) transplanting the cultured cell to the fallopian tube or uterus of the recipient female non-human mammal, allowing the cell to develop in the uterus of the female non-human mammal;
(d) identifying the germline transmission in the offspring genetically modified humanized non-human mammal of the pregnant female in step (c).

In some embodiments, the non-human mammal in the foregoing method is a mouse (e.g., a C57BL/6 mouse).

In some embodiments, the non-human mammal in step (c) is a female with pseudopregnancy (or false pregnancy).

In some embodiments, the fertilized eggs for the methods described above are C57BL/6 fertilized eggs. Other fertilized eggs that can also be used in the methods as described herein include, but are not limited to, FVB/N fertilized eggs, BALB/c fertilized eggs, DBA/1 fertilized eggs and DBA/2 fertilized eggs.

Fertilized eggs can come from any non-human animal, e.g., any non-human animal as described herein. In some embodiments, the fertilized egg cells are derived from rodents. The genetic construct can be introduced into a fertilized egg by microinjection of DNA. For example, by way of culturing a fertilized egg after microinjection, a cultured fertilized egg can be transferred to a false pregnant non-human animal, which then gives birth of a non-human mammal, so as to generate the non-human mammal mentioned in the methods described above.

In some embodiments, the method for generating a genetically modified animal comprises modifying the coding frame of the CFB gene of the non-human animal, e.g., by replacing the nucleic acid sequence (e.g., DNA or cDNA sequence) encoding the endogenous CFB with a nucleotide sequence encoding the human CFB under the control of endogenous regulatory elements of the CFB gene of the non-human animal. For example, one or more functional region sequences of the CFB gene of the non-human animal can be knocked out or inserted with a sequence, such that the endogenous CFB protein of the non-human animal is not expressed or is expressed at a lower level. In some embodiments, the modified coding frame of the CFB gene of the non-human animal can be all or portion of the nucleotide sequence of exons 1 to 18 of the CFB gene of the non-human animal.

In some embodiments, methods of making the genetically modified animal comprises inserting a nucleotide sequence encoding human or humanized CFB protein and/or auxiliary sequences after the endogenous regulatory element of the non-human animal's CFB gene. In some embodiments, the auxiliary sequence can be a stop codon, such that the CFB gene humanized animal model can express human or humanized CFB protein *in vivo,* but does not express CFB protein of non-human animal. In some embodiments, the auxiliary sequence includes WPRE (WHP Posttranscriptional Response Element), loxP, STOP, and/or polyA.

In some embodiments, a method of constructing a genetically modified non-human animal comprises using the aforementioned vectors, e.g., a targeting vector and/or a sgRNA vector.

In some embodiments, the method for making the genetically modified animal comprises:
(1) providing a plasmid comprising a human CFB gene fragment (e.g., a human CFB donor sequence), flanked by a 5' homologous arm and a 3' homologous arm, wherein the 5' and 3' homologous arms target an endogenous CFB gene;
(2) providing one or more small guide RNAs (sgRNAs) that target the endogenous CFB gene;
(3) modifying genome of a fertilized egg or an embryonic stem cell by using the plasmid of step (1), the sgRNAs of step (2), and Cas9;
(4) transplanting the fertilized egg obtained in step (3) into the oviduct of a pseudopregnant female non-human animal (e.g., a mouse) or transplanting the embryonic stem cell obtained in step (3) into a blastocyst which is then transplanted into the oviduct of a pseudopregnant female non-human animal (e.g., a mouse) to produce a child mouse that functionally expresses a humanized CFB protein; and

Preferably, further comprises step (5) mating the child non-human animal (e.g., a mouse) obtained in step (2) to obtain a homozygote non-human animal (e.g., a mouse).

In some embodiments, the fertilized egg is modified by CRISPR with sgRNAs that target a 5'-terminal targeting site and a 3'-terminal targeting site.

In some embodiments, the sequence encoding the humanized CFB protein is operably linked to an endogenous or human regulatory element at the endogenous CFB gene locus.

In some embodiments, the genetically-modified animal does not express an endogenous CFB protein.

In some embodiments, the method for making the genetically modified animal comprises:
(1) providing a plasmid comprising a human or chimeric CFB gene fragment (e.g., a human CFB donor sequence), flanked by a 5' homologous arm and a 3' homologous arm, wherein the 5' and 3' homologous arms target an endogenous CFB gene;
(2) providing one or more small guide RNAs (sgRNAs) that target the endogenous CFB gene; and
(3) modifying genome of a fertilized egg or an embryonic stem cell by inserting the human or chimeric CFB gene fragment (e.g., a human CFB donor sequence) into the genome.

### Methods of using genetically modified animals

Replacement of non-human genes in a non-human animal with homologous or orthologous human genes or human sequences, at the endogenous non-human locus and under control of endogenous or human regulatory elements (e.g., a promoter), can result in a non-human animal with qualities and characteristics that may be substantially different from a typical knockout-plus-transgene animal. In the typical knockout-plus-transgene animal, an endogenous locus is removed or damaged and a fully human transgene is inserted into the animal genome and presumably integrates at random into the genome. Typically, the location of the integrated transgene is unknown; expression of the human protein is measured by transcription of the human gene and/or protein assay and/or functional assay. Inclusion in the human transgene of upstream and/or downstream human sequences are apparently presumed to be sufficient to provide suitable support for expression and/or regulation of the transgene.

Genetically modified animals that express human or humanized CFB protein, e.g., in a physiologically appropriate manner, provide a variety of uses that include, but are not limited to, developing therapeutics for human diseases and disorders, and assessing the toxicity and/or the efficacy of these human therapeutics in the animal models.

The present disclosure also provides an application of a non-human animal modified by the CFB gene or a non-human animal obtained by any of the construction methods described above.

In some embodiments, the applications comprise:
A) the applications in product development involving human cells in immune processes related to CFB;
B) the applications as a model system related to CFB in pharmacological, immunological, microbiological, and medical research;
C) the applications of the production and use of animal experimental disease models in CFB related pathogenesis research and/or developing of diagnostic strategies and/or therapeutic strategies;
D) the applications in the screening, efficacy evaluation, validation, or assessment of human CFB signaling pathway modulators *in vivo;* or
E) the applications in studying the function of CFB genes, researching drugs and their efficacy targeting human CFB, and investigating immune-related disease drugs and anti-tumor drugs related to CFB.

The present disclosure provides a non-human animal expressing human or humanized CFB protein, which can be used for screening therapeutic agents (e.g., human CFB-specific modulators, oligonucleotide drugs, and/or polypeptide drugs). In some embodiments, the non-human animal is a model for human diseases, e.g., genetically induced diseases (knock-in or knock-out). In various embodiments, the genetically modified non-human animal also has a compromised immune system, e.g., genetically modified human tissue xenografts, including human solid tumors (e.g., breast cancer) or hematologic malignancies (e.g., lymphocytic tumors, B or T cell tumors).

In some embodiments, the genetically modified non-human animal can be used to determine the efficacy of therapeutic agents in treating various diseases. In some embodiments, the disease is related to CFB expression or abnormal expression (e.g., overexpression) of CFB. In some embodiments, the disease is one of which targeting CFB or down-regulating CFB expression is beneficial for treatment.

In some embodiments, the genetically modified non-human animal can be used to determine the efficacy of therapeutic agents (e.g., human CFB-specific modulators, oligonucleotide drugs, and/or polypeptide drugs) in treating various immune diseases. In some embodiments, the immune diseases comprise, but are not limited to, age-related macular degeneration (AMD), rheumatoid arthritis, colitis (comprising ulcerative colitis or Crohn's disease, e.g., perianal Crohn's disease), rheumatism, multiple sclerosis, Parkinson's disease, asthma, myasthenia gravis, or complement diseases, etc.

In some embodiments, the genetically modified non-human animal can be used to determine the efficacy of therapeutic agents (e.g., human CFB-specific modulators, oligonucleotide drugs, and/or polypeptide drugs) in treating various inflammatory infections. In some embodiments, the inflammation includes both acute inflammation and chronic inflammation. Specifically, it comprises, but is not limited to, IgA nephropathy, C3 glomerulopathy, glomerulonephritis, degenerative inflammation, exudative inflammation (e.g., serous inflammation, fibrinous inflammation, purulent inflammation, hemorrhagic inflammation, necrotizing inflammation, or catarrhal inflammation), proliferative inflammation, or specific inflammation (tuberculosis, syphilis, leprosy, lymphogranuloma, etc.).

In some embodiments, the genetically modified non-human animal can be used to determine whether an anti-CFB antibody or a CFB-specific oligonucleotide drug is an agonist or an antagonist. In some embodiments, the methods described herein can be used to detect the function of therapeutic agents (e.g., human CFB-specific modulators, oligonucleotide drugs, and/or polypeptide drugs), for example, whether a therapeutic agent can upregulate or downregulate an immune response, and/or whether the therapeutic agent can induce complement-mediated cytotoxicity (CMC) or antibody-dependent cellular cytotoxicity (ADCC). In some embodiments, the genetically modified non-human animal can be used to determine the effective dose of a therapeutic agent for treating a disease (e.g., an immune disease) in a subject.

The present disclosure further provides a method for determining the toxicity of therapeutic agents (e.g., human CFB-specific modulators, oligonucleotide drugs, and/or polypeptide drugs). The methods comprise administering the therapeutic agent to the non-human animal described above and evaluating changes in the animal body weight, red blood cell count, hematocrit, and/or hemoglobin. In some embodiments, the antibody can reduce red blood cells (RBCs), hematocrit, or hemoglobin by 20%, 30%, 40%, or more than 50%. In some embodiments, the body weight of the animal is at least 5%, 10%, 20%, 30%, or 40% less compared to the control group (e.g., the average body weight of animals not treated with the antibody).

The present disclosure further provides an animal model constructed by the methods described herein for developing products related to human cellular immune processes, producing human antibodies, or serving as a model system for pharmacological, immunological, microbiological, and medical research.

In some embodiments, an animal model generated by the methods described herein is provided for producing and utilizing animal experimental disease models of immune processes involving human cells, studying pathogens, or developing new diagnostic strategies and/or therapeutic strategies.

The present disclosure further provides an animal model generated by the methods described herein for screening, validating, evaluating, or studying the function of CFB gene, human CFB antibodies, drugs or efficacy targeting human CFB, and drugs for immune-related diseases and anti-tumor drugs.

### Non-human animal model with two or more human or chimeric (x) genes

The present disclosure further provides a method for constructing a transgenic animal model with two or more human or chimeric (x) genes. The animal model can comprise a human or chimeric CFB gene and a sequence encoding other human or chimeric (x) protein.

In some embodiments, the other human or chimeric (x) protein is at least one of CFD, C3, C5, C5AR1, ANGPTL3, IL36R, IGF1R, TSLP, TLR8, OX40, PD-1, PD-L1, and CTLA4. In some embodiments, the animal model described above further expresses at least one of human or humanized CFD, C3, C5, C5AR1, ANGPTL3, IL36R, IGF1R, TSLP, TLR8, OX40, PD-1, PD-L1, and CTLA4 protein.

The present disclosure further provides a method for constructing a non-human animal with two or more human or chimeric (x) genes, wherein the construction method comprises:
(1) providing the non-human animal described above, or the non-human animal obtained by the construction method described above;
(2) mating, performing *in vitro* fertilization, or directly genetically editing the non-human animal provided in step (1) with the other genetically modified non-human animal, followed by screening to obtain a multi-gene modified non-human animal.

In some embodiments, the other genetically modified non-human animal comprises a humanized non-human animal with one or a combination of two or more of the genes CFD, C3, C5, C5AR1, ANGPTL3, IL36R, IGF1R, TSLP, TLR8, OX40, PD-1, PD-L1, or CTLA4.

In some embodiments, the humanization of CFB is directly performed on a non-human animal already genetically modified to have human or chimeric CFD, C3, C5, C5AR1, ANGPTL3, IL36R, IGF1R, TSLP, TLR8, OX40, PD-1, PD-L1, and CTLA4 genes.

Each of the modified genes in the genome of the non-human animal or animal model with two or more human or chimeric genes is homozygous or heterozygous at the endogenous modified (preferably substitution) gene locus.

As these proteins may involve different mechanisms, a combination therapy that targets two or more of these proteins thereof may be a more effective treatment. In fact, many related clinical trials are in progress and have shown a good effect. The genetically modified animal model with two or more human or humanized genes can be used for determining effectiveness of a combination therapy that targets two or more of these proteins, e.g., an anti-CFB antibody, and an additional therapeutic agent for the treatment of cancer or immune diseases (e.g., asthma or atopic dermatitis). The method comprises administering to the non-human animal an anti-CFB antibody and the additional therapeutic agent, wherein the non-human animal has a tumor or immune disease, and determining the effect of the combination therapy on the immune tumor or immune disease. In some embodiments, the additional therapeutic agent is an antibody that specifically binds to CFD, C3, C5, C5AR1, ANGPTL3, IL36R, IGF1R, TSLP, TLR8, OX40, PD-1, PD-L1, and CTLA4.

In some embodiments, the additional therapeutic agent is an anti-CTLA4 antibody (e.g., ipilimumab), an anti-PD-1 antibody (e.g., nivolumab), or an anti-PD-L1 antibody. In some embodiments, the non-human animal described above further comprises a sequence encoding human or humanized PD-1, a sequence encoding human or humanized PD-L1, or a sequence encoding human or humanized CTLA-4. In some embodiments, the additional therapeutic agent is an anti-PD-1 antibody (e.g., nivolumab, pembrolizumab), an anti-PD-L1 antibody, or an anti-CTLA-4 antibody. In some embodiments, the aforementioned tumor includes one or more tumor cells expressing PD-L1 and/or PD-L2.

### DESCRIPTION OF DRAWINGS

Hereinafter, embodiments of the present disclosure are described in detail with reference to the accompanying drawings, wherein:
FIG. 1 is a schematic diagram showing mouse and human CFB gene locus (not to scale).
FIG. 2 is a schematic diagram of humanization of the mouse CFB gene locus (not to scale).
FIG. 3 is a schematic diagram showing an exemplary CFB targeting strategy with an exemplary targeting vector V1 design (not to scale).
FIG. 4 is a schematic diagram showing the FRT recombination process (not to scale).
FIG. 5 is a schematic diagram showing an exemplary CFB targeting strategy with an exemplary targeting vector V2 design (not to scale).
FIG. 6 show the PCR identification results of F1 generation CFB gene humanized mice, wherein primers used in A is WT-F/WT-R, primers used in B is WT-F/Mut-R, M is the marker, WT is the wild-type control, H2O is the water control, PC is the positive control.
FIG. 7 shows the Southern blot detection results of F1 generation CFB gene humanized mice, wherein WT is the wild-type control.
FIG. 8 shows the RT-PCR detection results, wherein +/+ is the wild-type C57BL/6 mouse, and H/H is the CFB gene humanized homozygous mouse, H2O is the water control.
FIG. 9 shows the ELISA detection results, wherein +/+ is the wild-type C57BL/6 mouse, and H/H is the CFB gene humanized homozygous mouse.
FIG. 10 shows the amino acid sequence of human CFB (NP_001701.2; SEQ ID NO: 2) and the amino acid sequence of mouse CFB (NP_032224.2; SEQ ID NO: 1).
FIG. 11 shows the amino acid sequence of human CFB (NP_001701.2; SEQ ID NO: 2) and the amino acid sequence of rat CFB (NP_997631.2; SEQ ID NO: 57).
FIG. 12 is ELISA detection results of human Ba protein expression in CFB humanized mice, wherein H/H is CFB gene humanized homozygous mice;
FIG. 13 is HE staining analysis of kidney tissues collected from CFB gene humanized mice;
FIG. 14A is determination of complement alternative pathway activity in groups treated with human C3b protein (71.4 µg/mL) and human C3b protein (71.4 µg/mL) + C5 protein (107 µg/mL), respectively, using a hemolysis assay, Nc is the solvent control.
FIG. 14B is determination of complement alternative pathway activity in groups treated with human C3b protein (71.4 µg/mL) + C5 protein (107 µg/mL) and human C3 protein (143 µg/mL) + human C5 protein (107 µg/mL), respectively, using a hemolysis assay, Nc is the solvent control.
FIG. 15 is determination of complement alternative pathway activity using the alternative pathway assay kit.
FIG. 16 is inhibition rate of the nucleic acid therapeutics on CFB protein (A) and mRNA (B) expression levels.
FIG. 17 is a schematic diagram of humanization of the mouse CFB gene locus (not to scale);
FIG. 18 is a schematic diagram showing an exemplary CFB targeting strategy with an exemplary targeting vector V3 design (not to scale).
FIG. 19 is a schematic diagram showing an exemplary CFB targeting strategy with an exemplary targeting vector V4 design (not to scale).

### EXAMPLES

The following describes the present disclosure in further detail with reference to specific embodiments, and the advantages and features of the present disclosure will become more apparent as the description proceeds. However, these embodiments are merely exemplary and do not limit the scope of the present disclosure in any way. Those skilled in the art should understand that the details and forms of the technical solutions of the present disclosure can be modified or replaced without departing from the spirit and scope of the present disclosure, but such modifications and replacements all fall within the protection scope of the present disclosure.

The following equipment and materials used in the following examples were obtained from companies identified below.

C57BL/6 mice were purchased from the National Institutes for Food and Drug Control, National Rodent Laboratory Animal Resources Center.

HUMAN Factor B ELISA KIT was purchased from Abcam, Cat. No: ab137973.

Unless otherwise indicated, the practice of the disclosure will employ conventional techniques of cell biology, cell culture, molecular biology, transgenic biology, microbiology, recombinant DNA, and immunology. These techniques are explained in detail in the following references. For example: Molecular Cloning A Laboratory Manual, 2nd Ed., ed. by Sambrook, Fritsch and Maniatis (Cold Spring Harbor Laboratory Press: 1989); DNA Cloning, Volumes I and II (D. N. Glover ed., 1985); Oligonucleotide Synthesis (M. J. Gait ed., 1984); Mullis et al. U.S. Pat. No. 4,683,195; Nucleic Acid Hybridization (B. D. Hames & S. J. Higgins eds. 1984); Transcription And Translation (B. D. Hames & S. J. Higgins eds. 1984); Culture Of Animal Cells (R. I. Freshney, Alan R. Liss, Inc., 1987); Immobilized Cells And Enzymes (IRL Press, 1986); B. Perbal, A Practical Guide To Molecular Cloning (1984); the series, Methods In ENZYMOLOGY (J. Abelson and M. Simon, eds.-in-chief, Academic Press, Inc., New York), specifically, Vols. 154 and 155 (Wu et al. eds.) and Vol. 185, "Gene Expression Technology" (D. Goeddel, ed.); Gene Transfer Vectors For Mammalian Cells (J. H. Miller and M. P. Calos eds., 1987, Cold Spring Harbor Laboratory); Immunochemical Methods In Cell And Molecular Biology (Mayer and Walker, eds., Academic Press, London, 1987); Handbook Of Experimental Immunology, Volumes I-V (D. M. Weir and C. C. Blackwell, eds., 1986); and Manipulating the Mouse Embryo, (Cold Spring Harbor Laboratory Press, Cold Spring Harbor, N.Y., 1986).

### Example 1: Method 1 of Constructing CFB Gene Humanized Mice

The mouse CFB gene (NCBI Gene ID: 14962, Primary source: MGI: 105975, UniProt: B8JJM5) is located at positions 35075350 to 5081492 on chromosome 17, NC_000083.7, based on transcript NM_008198.2 and encoded protein thereof NP_032224.2 (SEQ ID NO: 1). The human CFB gene (NCBI Gene ID: 629, Primary source: HGNC: 1037, UniProt: P00751-1) is located at positions 31946095 to 31952084 on chromosome 6, NC_000006.12, based on transcript NM_001710.6 and encoded protein thereof NP_001701.2 (SEQ ID NO: 2) is shown in **FIG. 1****.**

To achieve the objectives of the disclosure, a nucleotide sequence encoding a human CFB protein can be introduced at the endogenous mouse CFB gene locus, so that the mice express a human or humanized CFB protein. Specifically, using gene editing technology, under the control of a mouse CFB gene regulatory element, an approximately 5.8 kb nucleotide sequence comprising the coding region of a portion of exon 1 to a portion of exon 18 of the human CFB gene (e.g., from the start codon ATG to the stop codon TAA) is used to replace the corresponding approximately 5.7 kb nucleotide sequence comprising the coding region of a portion of exon 1 to a portion of exon 18 of the mouse CFB gene (e.g., from the start codon ATG to the stop codon TAA), resulting in a humanized CFB locus, the schematic diagram of which is shown in **FIG. 2****,** and achieving humanization of the mouse CFB gene.

Based on **FIG. 2****,** a targeting strategy shown in **FIG. 3** was further designed. The diagram shows that the targeting vector V1 comprises an upstream homology arm sequence, a downstream homology arm sequence of the mouse CFB gene, and an A fragment containing a human CFB fragment. Specifically, the upstream 5' homology arm sequence (SEQ ID NO: 3) is identical to the nucleotide sequence at position 35081089 to 35084696 of NCBI accession number NC_000083.7, and the downstream 3' homology arm sequence (SEQ ID NO: 4) is identical to the nucleotide sequence at position 35070668 to 35074536 of NCBI accession number NC_000083.7. The nucleotide sequence of the human CFB fragment (SEQ ID NO: 7) is identical to the nucleotide sequence at position 31946222 to 31952030 of NCBI accession number NC_000006.12. The upstream connection between the human CFB fragment sequence and the mouse sequence is designed as: 5'-AGGGCTTTGGGCCACTGGACTCTCTGGTGCTTTCCATGGGGAGCAATCTCAGCCCCC AACTCTGCCTGATGCCCTTTATCTTGGGC -3' (SEQ ID NO: 9), wherein the last "C" in the sequence "TTTCC" is the last nucleotide of the mouse sequence, and the "A" in the sequence "ATGGG" is the first nucleotide of the human sequence. The downstream connection between the human CFB fragment sequence and the mouse sequence is designed as: 5'-TGCTGCCCTGGCTGAAGGAGAAACTCCAAGATGAGGATTTGGGTTTTCTATAAAGAG CTTCCTGCAGGGAGAGTGTGAGGACAGATTAAAGCAGTTACA -3' (SEQ ID NO: 10), wherein the last "A" in the sequence "TATAA" is the last nucleotide of the human sequence, and the first "A" in the sequence "AGAGC" is the first nucleotide of the mouse sequence.

The targeting vector also comprises a resistance gene for positive clone screening, namely the neomycin phosphotransferase coding sequence (Neo), flanked by two site-specific recombination system Frt recombination sites arranged in the same direction, forming a Neo cassette. Specifically, the connection between the Neo cassette upstream and the mouse CFB gene is designed as 5'-GGGGTTGTTCTTAGCTTGGACTCTTCTTCATGGCTCCTTTGCTGCAGACGTCGACGGT ATCGATAAGCTTGATATCGAATTCCGAAGTTC-3' (SEQ ID NO: 11), wherein the last "C" in the sequence "CAGAC" is the last nucleotide of the mouse CFB gene, and the first "G" in the sequence "GTCG" is the first nucleotide of the Neo cassette; the connection between the Neo cassette downstream and the mouse CFB gene is designed as 5'-TATAGGAACTTCATCAGTCAGGTACATAATGGTGGATCCTTAACTTGGGCCTAGCAAG CCAGGACTGCTGGAGGT-3' (SEQ ID NO: 12), wherein the last "C" in the sequence "GATCC" is the last nucleotide of the Neo cassette, and the first "T" in the sequence "TTAAC" is the first nucleotide of the mouse CFB gene. The mRNA sequence of the engineered humanized mouse CFB is shown in SEQ ID NO: 8, and the expressed protein sequence is shown in SEQ ID NO: 2.

The construction of the targeting vector was carried out using conventional methods, such as enzyme digestion and ligation. After preliminary verification of the constructed targeting vector by enzyme digestion, the targeting vector was sent to a sequencing company for sequencing. The targeting vector with the correct sequence was then electroporated into the embryonic stem cells of C57BL/6 mice. The obtained cells were screened using the positive clone selection marker gene (Neo) to select the correct positive clone cells. The selected positive clone cells (black mice) were introduced into isolated blastocysts (white mice). The resulting chimeric blastocysts were briefly cultured in a culture medium and then transplanted into the fallopian tubes of recipient female mice (white mice) to produce F0 generation chimeric mice (black and white). The F0 generation chimeric mice were backcrossed with wild-type mice to obtain F1 generation mice, and the F1 heterozygous mice were interbred to produce F2 generation homozygous mice. Positive mice were also mated with Flp tool mice to remove the positive clone selection marker gene (a schematic diagram of this process is shown in FIG. 4), and subsequent interbreeding produced CFB gene humanized homozygous mice..

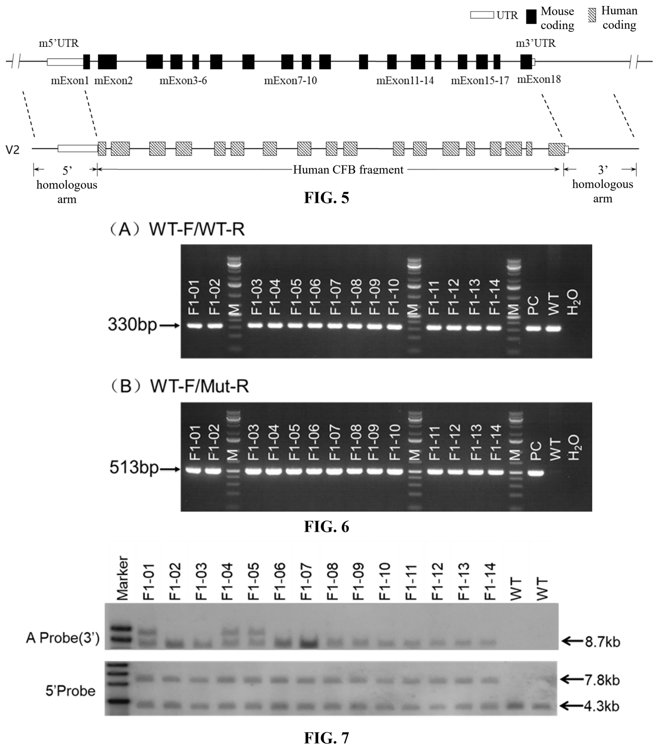

Additionally, CRISPR/Cas9 technology was used for gene editing to further design targeting strategies.. **FIG. 5** shows another exemplary targeting strategy. The figure displays the targeting vector V2 with upstream homologous arm and downstream homologous arm sequences at the mouse CFB gene, along with a human CFB fragment. A schematic diagram of the engineered humanized CFB gene locus is shown in **FIG. 2****.** Specifically, the upstream 5' homologous arm sequence (SEQ ID NO: 5) is identical to the nucleotide sequence at position 35081089 to 35082539 of NCBI accession number NC_000083.7, and the downstream 3' homologous arm sequence (SEQ ID NO: 6) is identical to the nucleotide sequence at position 35073826 to 35075402 of NCBI accession number NC_000083.7. The nucleotide sequence of the human CFB fragment (SEQ ID NO: 7) is identical to the nucleotide sequence at position 31946222 to 31952030 of NCBI accession number NC_000006.12. The mRNA sequence of the engineered humanized CFB mouse is shown in SEQ ID NO: 8, and the expressed protein sequence is shown in SEQ ID NO: 2.

The construction of the targeting vector was carried out using conventional methods, e.g., enzyme digestion and ligation, or direct synthesis. The constructed targeting vector is preliminarily verified by enzyme digestion and then sent to a sequencing company for sequencing verification. The sequenced targeting vector with correct sequence is used for subsequent experiments.

The target sequence determines the targeting specificity of sgRNA and the efficiency of Cas9-induced gene cutting. Therefore, the selection and design of highly specific target sequences are prerequisites for constructing sgRNA expression vectors. The sgRNA sequence recognizing the target site is designed and synthesized. An exemplary sgRNA target sequence in the CFB gene is as follows:
sgRNA1 target site (SEQ ID NO: 13): 5'-AGCCTAAGACCAAGAGGACGAGG-3';
sgRNA2 target site (SEQ ID NO: 14): 5'-GTGGAAGTCCCGGGCATAAGAGG-3'.

The activity of sgRNA is determined using the UCA kit to confirm its ability to mediate efficient cutting. After adding enzyme cutting sites to the 5' end and complementary strand, respectively, to obtain the forward and reverse oligonucleotide sequences as shown in Table 5, the annealed product is ligated into the pT7-sgRNA plasmid (linearized with BbsI), obtaining the expression vectors pT7-CFB-1 and pT7-CFB-2.

**Table 5. Sequences of sgRNA1 and sgRNA2**

| sgRNA1 sequences | |
|---|---|
| SEQ ID NO: 15 | Upstream: 5'-AGCCTAAGACCAAGAGGACG-3' |
| SEQ ID NO: 16 (forward oligonucleotide) | Upstream: 5'-TAGGAGCCTAAGACCAAGAGGACG-3' |
| SEQ ID NO: 17 | Downstream: 5'-CGTCCTCTTGGTCTTAGGCT-3' |
| SEQ ID NO: 18 (reverse oligonucleotide) | Downstream: 5'-AAACCGTCCTCTTGGTCTTAGGCT-3' |

| sgRNA2 sequences | |
|---|---|
| SEQ ID NO: 19 | Upstream: 5'-GTGGAAGTCCCGGGCATAAG-3' |
| SEQ ID NO: 20((forward oligonucleotide) | Upstream: 5'-TAGGGTGGAAGTCCCGGGCATAAG-3' |
| SEQ ID NO: 21 | Downstream: 5'-CTTATGCCCGGGACTTCCAC-3' |
| SEQ ID NO: 22 (reverse oligonucleotide) | Downstream: 5'-AAACCTTATGCCCGGGACTTCCAC-3' |

The pT7-sgRNA vector was synthesized through a plasmid synthesis company, which included a DNA fragment containing the T7 promoter and sgRNA scaffold (SEQ ID NO: 23), and was ligated to the backbone vector (Takara, Cat. No: 3299) after restriction enzyme digestion (EcoRI and BamHI). The resulting plasmid was confirmed by sequencing. The pre-mixed Cas9 mRNA, the targeting vector, and *in vitro* transcription products of the pT7-CFB-1 and pT7-CFB-2 plasmids (using Ambion^{™} *in vitro* transcription kit to carry out the transcription according to the method provided in the product instruction) were injected into the cytoplasm or nucleus of mouse fertilized eggs (e.g., C57BL/6 mice) with a microinjection instrument. The microinjection of fertilized eggs was carried out according to the method described, e.g., in A. Nagy, et al., "Manipulating the Mouse Embryo: A Laboratory Manual (Third Edition)," Chemistry Industry Press, 2006. The injected fertilized eggs were briefly cultured in a medium and then transplanted into the fallopian tubes of the recipient female mice for development. The resulting mice (F0 generation) were bred through cross-breeding and self-breeding to expand the population and establish a stable CFB gene humanized mouse line.

The genotype of somatic cells in F1 generation mice can be identified using the PCR method (primers as shown in Table 6). Exemplary identification results for F1 generation mice are shown in **FIG. 6****,** wherein the 14 mice numbered F1-01 to F1-14 were all positive mice.

**Table 6: PCR primer sequences and recombinant fragment size**

| Primers | SEQ ID NO | Primer sequences (5'-3') | Recombinant fragment size |
|---|---|---|---|
| WT-F | SEQ ID NO: 24 | CTGCAGATGCCAGAGCAGATTGC | 330bp |
| WT-R | SEQ ID NO: 25 | CAAGCACTGGAGTTGCGCTCACACC | |
| WT-F | SEQ ID NO: 24 | CTGCAGATGCCAGAGCAGATTGC | 513bp |
| Mut-R | SEQ ID NO: 26 | GGTCTTGAGTCTTCAGGGTGCTCCA | |

The positive F1 generation mice identified by PCR were then verified by Southern Blot. Genomic DNA of the positive F1 generation mice was extracted from mouse tails, digested with BglII or ScaI enzyme, respectively, transferred membrane, and then hybridized. The 5' probe (5'Probe) and the 3' probe (A Probe(3')) are located at the 5' homologous arm and at the human CFB fragment, respectively (specific probes and target fragment lengths are shown in Table 7). As shown in **FIG. 7****,** based on the PCR and sequencing results, the results indicate that 8 mice numbered F1-03 and F1-08 to F1-14 were positive mice. This indicates that the method described herein can construct CFB gene humanized mice that can be stably passed on without random insertion.

**Table 7 Specific probes and target fragment lengths**

| Restriction enzyme | Probe | WT size | Recombinant fragment size |
|---|---|---|---|
| BglII | A Probe(3') | -- | 8.7kb |
| ScaI | 5'Probe | 4.3kb | 7.8kb |

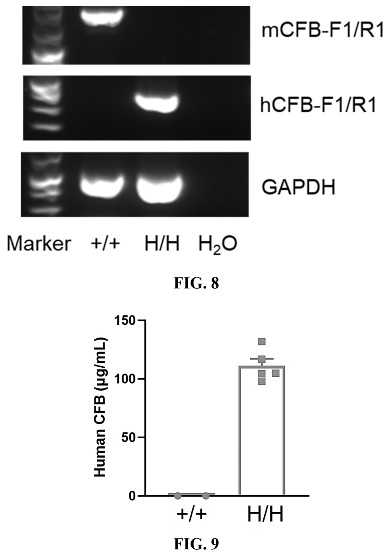

5'Probe-F (SEQ ID NO: 27): 5'-GCTCACTGCTTCCATGACATTCAG-3',
5'Probe-R (SEQ ID NO: 28): 5'-GGGTTCAGGCACCTGGCATGGGTGG-3';
A Probe (3')-F (SEQ ID NO: 29): 5'-CTGGGGAGATGCCAAGTGGTCAGC-3',
A Probe (3')-R (SEQ ID NO: 30): 5'-AGCTGATTACACCAACCTGCAGA-3';
The expression of mRNA in CFB gene humanized mice was detected by RT-PCR. Specifically, one 8-week-old male C57BL/6 mouse (+/+) and one 8-week-old male CFB gene humanized homozygous (H/H) mouse prepared in this example were selected. After euthanasia by cervical dislocation, liver tissues were collected, and RT-PCR detection was performed using the primer sequences shown in the table below. The results are shown in **FIG. 8. FIG. 8** shows that only mouse CFB mRNA was detected in the wild-type C57BL/6 mouse, and no human CFB mRNA was detected. In the CFB gene humanized homozygous mouse, only human CFB mRNA was detected.

**Table 8. RT-PCR primer sequences and target fragment size**

| Primer | SEQ ID NO | Sequence (5'-3') | Fragment size |
|---|---|---|---|
| mCFB-F1 | SEQ ID NO: 31 | CTCTGGTGGACTCCGTGAAC | 867bp |
| mCFB-R1 | SEQ ID NO: 32 | CTCCCCAGCTAATCACACCA | |
| hCFB-F1 | SEQ ID NO: 33 | TCCAACTACCACTTGCCAGC | 400bp |
| hCFB-R1 | SEQ ID NO: 34 | GATGTGAAAGTCTCGGGCGT | |
| GAPDH-F | SEQ ID NO: 35 | TCACCATCTTCCAGGAGCGAGA | 479bp |
| GAPDH-R | SEQ ID NO: 36 | GAAGGCCATGCCAGTGAGCTT | |

Furthermore, the expression of human CFB protein in CFB humanized mice was detected by conventional methods such as ELISA. Specifically, five 8-week-old male C57BL/6 mice (+/+) and five 8-week-old male CFB gene humanized homozygous (H/H) mice prepared in this example were selected. Serum was collected, and the detection results using the Human Factor B ELISA Kit (Abcam, Cat. No: ab137973, detection range: 2.188 ng/ml-140 ng/ml) are shown in **FIG. 9****.** Based on the RT-PCR results, this indicates that the CFB gene humanized homozygous mice successfully express the human CFB protein.

### Example 2: Functional Analysis of CFB Humanized Mice

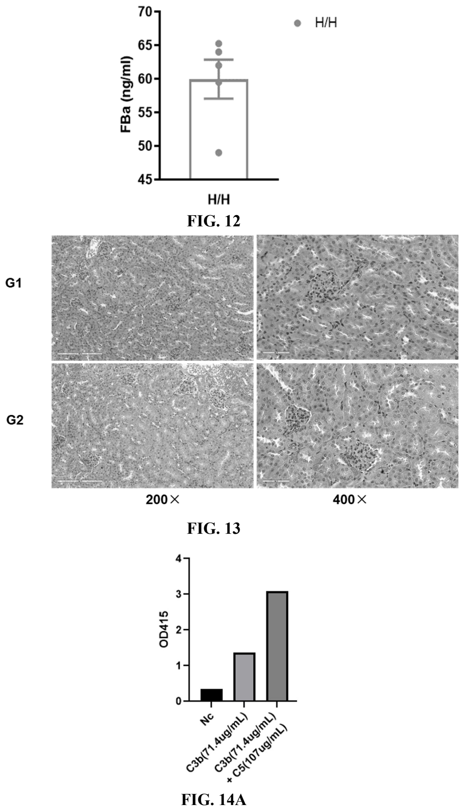

The complement factor B encoded by the CFB gene is a component of the alternative pathway of complement activation. Complement factor B circulates in the blood as a single-chain polypeptide. Upon activation of the alternative pathway, it can be cleaved by complement factor D to generate the non-catalytic chain Ba (also known as Fba) and the catalytic subunit Bb. Further, the expression of human Ba protein in the CFB humanized mice prepared in Example 1 was detected by ELISA. Specifically, CFB gene humanized homozygous (H/H) mice were selected, serum was collected, and detection was performed using a human-mouse cross-reactive ELISA kit (Ba ELISA Kit, QuidelOrtho, Cat. No: A033). The results are shown in **FIG. 12****.**

As shown in **FIG. 12****,** Ba protein is detected in the CFB humanized homozygous mice when using the Ba ELISA Kit. This indicates that the human CFB protein in the CFB gene humanized homozygous mice can be cleaved by CFD to generate the Ba fragment, and the CFB signaling pathway functions normally in these mice.

In another experiment, six 7-week-old male wild-type C57BL/6 mice (+/+) and six CFB humanized homozygous mice (H/H) were selected, and peripheral blood was collected for blood biochemical analysis. The blood biochemical indices comprise urea (UREA), serum creatinine (CREA), and total protein (TP). The results are shown in Table 9. The results indicate that the CFB gene humanization did not affect the levels of UREA, CREA, and TP in the mice, and the modified mice exhibited renal function status identical to that of wild-type mice.

**Table 9: results of blood biochemical analysis**

| Group | UREA (mmol/L) | CREA (mmol/L) | TP (g/L) |
|---|---|---|---|
| +/+ | 5.71 ± 0.29 | 2.77 ± 0.20 | 54.76 ± 0.67 |
| H/H | 5.71 ± 0.51 | 3.5 ± 0.48 | 53.25 ± 0.83 |

Furthermore, three 7-week-old male wild-type C57BL/6 mice (+/+) and three CFB humanized homozygous mice (H/H) were selected. After euthanasia by cervical dislocation, kidney tissues were collected for HE staining and analysis. The results are shown in **FIG. 13****.** No damage was observed in the kidney tissues of mice in the G1 (C57BL/6 mice) group and the G2 (CFB humanized homozygous mice) group, indicating that CFB gene humanization does not affect kidney health in mice.

Furthermore, the hemolytic activity of the complement alternative pathway (OD415 when hemolysis occurs) in CFB humanized mice was determined. Specifically, three male CFB humanized homozygous mice (H/H) were selected. After euthanasia by cervical dislocation, serum was collected, added with rabbit erythrocyte separation solution and mixed, and the mixture was divided into 4 groups. Wherein the control group (Nc) was added with an equal volume of H₂O or PBS, while the experimental groups were added with human C3b protein (71.4 µg/mL) or human C3b protein (71.4 µg/mL) + C5 protein (107 µg/mL). The OD415 values were obtained with a spectrophotometer to determine the serum complement alternative pathway activity. In another similar experiment, serum collected from CFB humanized homozygous mice was divided into 3 groups. Wherein the control group (Nc) was added with an equal volume of H₂O or PBS, while the experimental groups were added with human C3b protein (71.4 µg/mL) + C5 protein (107 µg/mL), or human C3 protein (143 µg/mL) + human C5 protein (107 µg/mL). The OD415 values were obtained with a spectrophotometer to determine the serum complement alternative pathway activity.

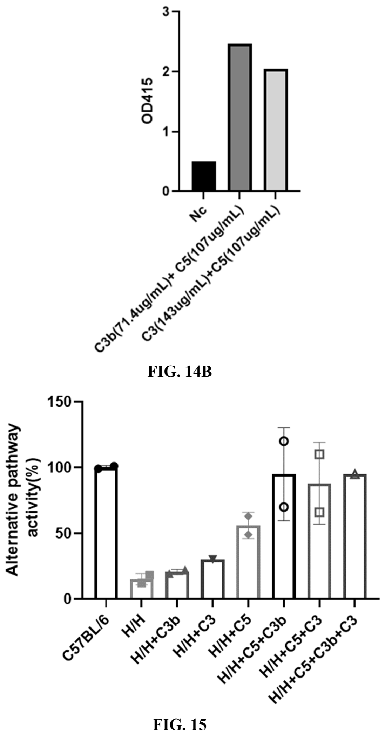

The results are shown in **FIG. 14A** and **FIG. 14B****.** Compared with the control group, the supplement of human C3b+C5 and C3+C5 recombinant proteins can rescue the alternative pathway.

The complement alternative pathway activation activity in CFB humanized mice (homozygous H/H) was detected using the Alternative Pathway ELISA Kit (Hycult Biotech, Cat. No: HIT422). Specifically, 5-6-week-old humanized CFB male mice were selected. After euthanasia by cervical dislocation, serum was collected, divided into 7 groups and added with C3b, C3, C5, C3b+C5, or C3+C5 recombinant proteins, respectively, and the complement alternative pathway activity with different components supplemented was detected using the Alternative Pathway ELISA Kit. The results showed in **FIG. 15** indicate that the supplement of C3b+C5 or C3+C5 can rescue the complement alternative pathway activity.

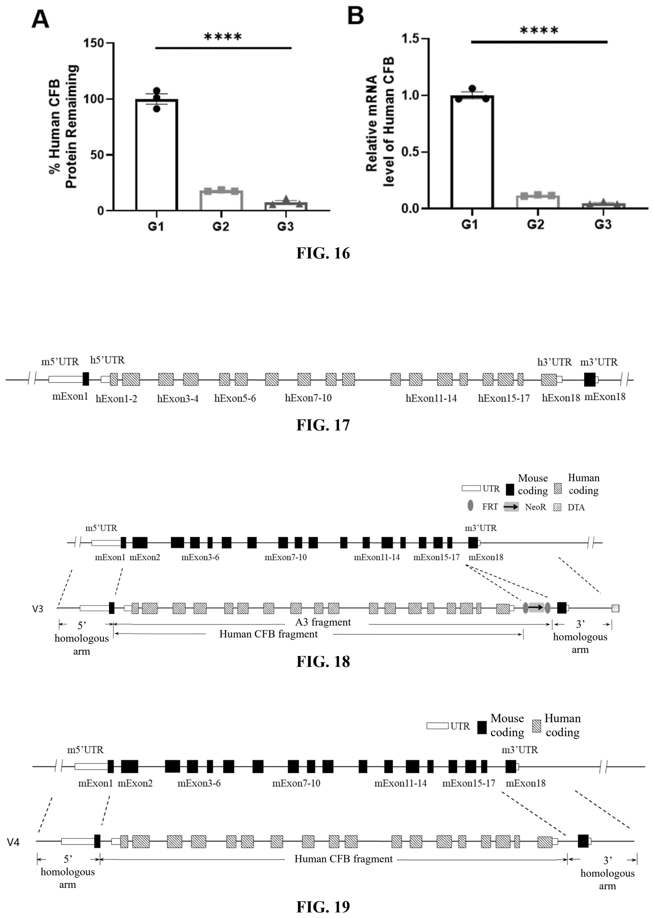

Humanized CFB mice (8-week-old, male, homozygous H/H) were randomly divided into a control group or treatment groups (n=3/group). On the grouping day (day 0), the treatment groups were randomly injected subcutaneously with 1 mpk (G2) or 3 mpk (G3) of an siRNA therapeutic (sense strand is SEQ ID NO: 58 conjugated with N-[tris(GalNAc-alkyl)-amidodecanoyl]-4-hydroxyprolinol to 3' end thereof; antisense strand as shown in SEQ ID NO: 59). The control group (G1) was injected with an equal volume of PBS vehicle. On day 7 after grouping, liver tissues and plasma of the mice were collected to detect human CFB mRNA levels and protein levels, respectively. The results indicate that the plasma concentration of human CFB protein in the treatment groups (G2, G3) decrease significantly compared with the control group (G1) **(****FIG. 16A****);** the human CFB mRNA levels in the liver tissues of the treatment groups (G2, G3) also decrease significantly (**FIG. 16B**) in a dose-dependent manner. This indicates that humanized CFB mice can be used for preclinical drug screening of nucleic acid therapeutics.
SEQ ID NO: 58: sense (5'-3') Gm-s-Am-s-Am-Um-Um-Cm-Cm-Um-Gf-Af-Af-Um-Um-Um-Um-Am-Um-Gm-Am-Cm-Um
SEQ ID NO: 59: antisense (5'-3') Am-s-dG-s-Um-Cm-dA-Um-dA-Am-Am-Am-Um-dT-Cm-Af-Gm-Gm-Am-Am-Um-Um-Cm-s-Cm-s-Um

Wherein, m is 2'-O-methyl, s is phosphorothioate linkage, f is 2'-fluoro modification, dG is 2'-deoxyguanosine-3'-phosphate, dA is 2'-deoxyadenosine-3'-phosphate, and dT is 2'-deoxythymidine-3'-phosphate.

### Example 3: Method 2 of Constructing CFB Gene Humanized Mice

The mouse CFB gene (NCBI Gene ID: 14962, Primary source: MGI: 105975, UniProt: B8JJM5, located at positions 35075350 to 5081492 on chromosome 17 NC_000083.7, based on transcript NM_008198.3 and encoded protein thereof NP_032224.2 (SEQ ID NO: 1)) and the human CFB gene (NCBI Gene ID: 629, Primary source: HGNC: 1037, UniProt: P00751-1, located at positions 31946095 to 31952084 on chromosome 6 NC_000006.12, based on transcript NM_001710.6 and encoded protein thereof NP_001701.2 (SEQ ID NO: 2)).

Futhermore, to achieve the objectives of the disclosure, an approximately 13.0 kb nucleotide sequence comprising the upstream sequence of the 5'UTR to the downstream sequence of the 3'UTR of the human CFB gene can be used to replace an approximately 5.4 kb nucleotide sequence comprising a portion of exon 1 to exon 17 of the mouse CFB gene, resulting in a humanized CFB locus, the schematic diagram of which is shown in **FIG. 17****,** and achieving humanization of the mouse CFB gene.

Based on **FIG. 17****,** a targeting strategy shown in **FIG. 18** was further designed. The targeting vector V3 (shown in **FIG. 18**) comprises an upstream homologous arm sequence, a downstream homologous arm sequence of the mouse CFB gene, and an A3 fragment comprising a human CFB fragment. Specifically, the upstream 5' homologous arm sequence (SEQ ID NO: 37) is identical to the nucleotide sequence at position 35081039 to 35084904 of NCBI accession number NC_000083.7, and the downstream 3' homologous arm sequence (SEQ ID NO: 38) is identical to the nucleotide sequence at position 35071591 to 35075667 of NCBI accession number NC_000083.7. The nucleotide sequence of the human CFB gene fragment (SEQ ID NO: 39) is identical to the nucleotide sequence at position 31940095 to 31953084 of NCBI accession number NC_000006.12. The upstream connection between the human CFB fragment sequence and the mouse sequence is designed as: 5'-TGACAATGGAGAGCCCCCAGCTCTGCCTCGTCCTCTTGGTCTTAGGCTTAATTATTAT TTTTAATCAACAGCTTTAGACAGAGAACCTTGGTTT-3' (SEQ ID NO: 41), wherein the last "T" in the sequence "GGCTT" is the last nucleotide of the mouse sequence, and the first "A" in the sequence "AATTA" is the first nucleotide of the human sequence. The downstream connection between the human CFB fragment sequence and the mouse sequence is designed as: 5'-GATCTCTGTGCCTCAACACTGCTGGCTACTCCCTCTTTCTCGAAGTTCCTATTCTCTA GAAAGTATAGGAACTTCAGGTCTGAAGA-3' (SEQ ID NO: 42), wherein the last "C" in the sequence "TTCTC" is the last nucleotide of the human sequence, and the first "G" in the sequence "GAAGT" is the first nucleotide of the mouse sequence.

The targeting vector further comprises a resistance gene for positive clone screening, namely the neomycin phosphotransferase coding sequence (Neo), flanked by two site-specific recombination system Frt recombination sites arranged in the same direction, forming a Neo cassette. The upstream connection between the Neo cassette and the human CFB gene is designed as: 5'-CCATCCTGTGATCTCTGTGCCTCAACACTGCTGGCTACTCCCTCTTTCTCGAAGTTCC TATTCTCTAGAAAGTATAGGAACTTCA-3' (SEQ ID NO: 43), wherein the last "C" in the sequence "TTTCTC" is the last nucleotide of the human CFB gene, and the first "G" in the sequence "GAAGT" is the first nucleotide of the Neo cassette. The downstream connection between the Neo cassette and the mouse CFB gene is designed as: 5'-GAAGTTCCTATTCTCTAGAAAGTATAGGAACTTCCAGAAGACAGGGGGAGCTAGGC TCCCAGTGCACTTCTTTAG-3' (SEQ ID NO: 44), wherein the last "C" in the sequence "ACTTC" is the last nucleotide of the Neo cassette, and the "C" in the sequence "CAGAA" is the first nucleotide of the mouse CFB gene. The mRNA sequence of the engineered humanized mouse CFB is shown in SEQ ID NO: 40, and the expressed protein sequence is shown in SEQ ID NO: 2.

The construction of the targeting vector was carried out using conventional methods, such as enzyme digestion and ligation. After preliminary verification of the constructed targeting vector by enzyme digestion, the targeting vector was sent to a sequencing company for sequencing. The targeting vector with the correct sequence was then electroporated into the embryonic stem cells of C57BL/6 mice. The obtained cells were screened using the positive clone selection marker gene (Neo) to select the correct positive clone cells. The selected positive clone cells (black mice) were introduced into isolated blastocysts (white mice). The resulting chimeric blastocysts were briefly cultured in a culture medium and then transplanted into the fallopian tubes of recipient female mice (white mice) to produce F0 generation chimeric mice (black and white). The F0 generation chimeric mice were backcrossed with wild-type mice to obtain F1 generation mice, and the F1 heterozygous mice were interbreded to obtain F2 homozygous mice. Positive mice were also mated with Flp tool mice to remove the positive clone selection marker gene, and then interbred to obtain CFB gene humanized homozygous mice.

Additionally, gene editing can also be performed using the CRISPR/Cas9 system, further designing targeting vector V4 (shown in **FIG. 19**), which comprises upstream homologous arm and downstream homologous arm sequences at the mouse CFB gene, along with a human CFB fragment. A schematic diagram of the engineered humanized CFB gene locus is shown in **FIG. 17****.** Specifically, the upstream 5' homologous arm sequence (SEQ ID NO: 45) is identical to the nucleotide sequence at position 35081039 to 35082302 of NCBI accession number NC_000083.7, and the downstream 3' homologous arm sequence (SEQ ID NO: 46) is identical to the nucleotide sequence at position 35074350 to 35075667 of NCBI accession number NC_000083.7. The nucleotide sequence of the human CFB fragment (SEQ ID NO: 39) is identical to the nucleotide sequence at position 31940095 to 31953084 of NCBI accession number NC_000006.12. The mRNA sequence of the engineered humanized CFB mouse is shown in SEQ ID NO: 40, and the expressed protein sequence is shown in SEQ ID NO: 2.

The construction of the targeting vector was carried out using conventional methods, e.g., enzyme digestion and ligation, or direct synthesis. The constructed targeting vector is preliminarily verified by enzyme digestion and then sent to a sequencing company for sequencing verification. The sequenced targeting vector with correct sequence is used for subsequent experiments.

The target sequence determines the targeting specificity of sgRNA and the efficiency of Cas9-induced gene cutting. Therefore, the selection and design of highly specific target sequences are prerequisites for constructing sgRNA expression vectors. The sgRNA sequence recognizing the target site is designed and synthesized. An exemplary sgRNA target sequence in the CFB gene is as follows:
sgRNA3 target site (SEQ ID NO: 47): 5'-TGCAGGTTCGAGTCTGCACGGGG-3';
sgRNA4 target site (SEQ ID NO: 48): 5'-TCTTCTACAGGAGATTCCGGGGG-3'.

The activity of sgRNA is determined using the UCA kit to confirm its ability to mediate efficient cutting. After adding enzyme cutting sites to the 5' end and complementary strand, respectively, to obtain the forward and reverse oligonucleotide sequences as shown in Table 10, the annealed product is ligated into the pT7-sgRNA plasmid (linearized with BbsI), obtaining the expression vectors pT7-CFB-1 and pT7-CFB-2.

**Table 10. Sequences of sgRNA3 and sgRNA4**

| sgRNA3 sequences | |
|---|---|
| SEQ ID NO: 49 | Upstream: 5'-TGCAGGTTCGAGTCTGCACG-3' |
| SEQ ID NO: 50 (forward oligonucleotide) | Upstream: 5'-TAGGTGCAGGTTCGAGTCTGCACG-3' |
| SEQ ID NO: 51 | Downstream: 5'-CGTGCAGACTCGAACCTGCA-3' |
| SEQ ID NO: 52 (reverse oligonucleotide) | Downstream: 5'-AAACCGTGCAGACTCGAACCTGCA-3' |

| sgRNA4 sequences | |
|---|---|
| SEQ ID NO: 53 | Upstream: 5'-TCTTCTACAGGAGATTCCGG-3' |
| SEQ ID NO: 54((forward oligonucleotide) | Upstream: 5'-TAGGTCTTCTACAGGAGATTCCGG-3' |
| SEQ ID NO: 55 | Downstream: 5'-CCGGAATCTCCTGTAGAAGA-3' |
| SEQ ID NO: 56 (reverse oligonucleotide) | Downstream: 5'-AAACCCGGAATCTCCTGTAGAAGA-3' |

The pT7-sgRNA vector was synthesized through a plasmid synthesis company, which included a DNA fragment containing the T7 promoter and sgRNA scaffold (SEQ ID NO: 23), and was ligated to the backbone vector (Takara, Cat. No: 3299) after restriction enzyme digestion (EcoRI and BamHI). The resulting plasmid was confirmed by sequencing. The pre-mixed Cas9 mRNA, the targeting vector, and *in vitro* transcription products of the pT7-CFB-1 and pT7-CFB-2 plasmids (using Ambion^{™} *in vitro* transcription kit to carry out the transcription according to the method provided in the product instruction) were injected into the cytoplasm or nucleus of mouse fertilized eggs (e.g., C57BL/6 mice) with a microinjection instrument. The microinjection of fertilized eggs was carried out according to the method described, e.g., in A. Nagy, et al., "Manipulating the Mouse Embryo: A Laboratory Manual (Third Edition)," Chemistry Industry Press, 2006. The injected fertilized eggs were briefly cultured in a medium and then transplanted into the fallopian tubes of the recipient female mice for development. The resulting mice (F0 generation) were bred through cross-breeding and self-breeding to expand the population and establish a stable CFB gene humanized mouse line.

### EXAMPLE 4: In vivo efficacy verification

The CFB humanized mice disclosed in this invention can be used to evaluate the efficacy of modulators targeting human CFB. For example, CFB humanized homozygous mice are selected and randomly divided into a control group and a treatment group. On day 0 after grouping, the treatment group is injected with a drug targeting human CFB, while the control group is injected with an equal volume of PBS. On day 14 after administration, peripheral blood is collected to detect the expression level of human CFB protein in the mouse serum. Meanwhile on day 14, the mice are euthanized, liver tissues are collected, and the mRNA expression level in the mouse liver is detected. During the experiment, the body weight of the mice is regularly measured. Based on the mouse body weight data and the expression level of human CFB protein in the mice, the *in vivo* efficacy and safety of the drug in the humanized CFB mice can be effectively evaluated.

### EXAMPLE 5: Generation of multi-gene humanized mice

The methods described herein or the CFB gene humanized mice produced by the disclosed methods can also be used to create multi-humanized mouse models. For example, in Example 1, the embryonic stem cells used for microinjection can be obtained from mice containing at least one gene modification e.g., modified CFD, C3, C5, C5AR1, ANGPTL3, IL36R, IGF1R, TSLP, TLR8, OX40, PD-1, PD-L1, CTLA4. Alternatively, based on the humanized CFB mice, double or multi-humanized mouse models can be obtained using isolated mouse embryonic stem cells and gene recombination targeting techniques. CFB homozygous or heterozygous mice obtained by the methods described herein can also be crossed with other gene-modified mice. Their offspring can be screened, and according to Mendelian inheritance, there is a certain probability of obtaining multi-gene modified mice with humanized CFB gene and other gene modifications. Interbreeding these heterozygous mice can produce homozygous mice with double or multiple gene modifications.

The preferred embodiments of the present disclosure have been described in detail above. However, the present disclosure is not limited to the specific details in the embodiments described above. Within the scope of the technical concept of the present disclosure, various simple modifications can be made to the technical solutions of the present disclosure, and all such simple modifications fall within the protection scope of the present disclosure.

Additionally, it should be noted that the various specific technical features in the embodiments described above can be combined in any suitable way without conflict. To avoid unnecessary repetition, the present disclosure does not separately describe all possible combinations. Furthermore, the various different embodiments of the present disclosure can also be combined arbitrarily, as long as such combinations do not contradict the spirit of the present disclosure. These combinations should also be considered as part of the disclosed content of the present disclosure.

## Claims

1. A genetically-modified, non-human animal whose genome comprises at least one chromosome comprising a sequence encoding a human or chimeric complement factor B (CFB).

2. The animal of claim 1, wherein the sequence encoding the human or chimeric CFB is regulated by regulatory elements, and the regulatory element is an endogenous regulatory element or a human regulatory element.

3. The animal of claim 1, wherein the chimeric CFB is a humanized CFB, and the humanized CFB comprises a portion of a human CFB; preferably, the portion of the human CFB comprises the Ba region and/or the Bb region; further preferably, the Bb region comprises a VWA domain; further preferably, the Bb region comprises von Willebrand factor and/or peptidase S1.

4. The animal of claim 3, wherein the humanized CFB comprises at least 50 to 764 contiguous amino acid sequences that are identical to a portion of the human CFB.

5. The animal of any one of claims 1-4, wherein the animal is a mammal, such as a monkey or a rodent (e.g., a mouse or a rat).

6. The animal of any one of claims 1-5, wherein the human or chimeric CFB comprises an amino acid sequence set forth in SEQ ID NO: 2 or amino acids 26-764 of SEQ ID NO: 2, or an amino acid sequence that is at least 70%, 75%, 80%, 85%, 90%, 95%, or 99% identical to SEQ ID NO: 2 or amino acids 26-764 of SEQ ID NO: 2, or an amino acid sequence that differs from SEQ ID NO: 2 or amino acids 26-764 of SEQ ID NO: 2 by no more than 10, 9, 8, 7, 6, 5, 4, 3, 2, or 1 amino acid, or an amino acid sequence comprising a substitution, a deletion and/or insertion of one or more amino acid residue set forth in SEQ ID NO: 2 or amino acids 26-764 of SEQ ID NO: 2.

7. The animal of any one of claims 1-6, wherein the animal does not express endogenous CFB or expresses a decreased level of endogenous CFB as compared to CFB expression level in a wild-type animal.

8. A genetically-modified, non-human animal, wherein at the endogenous CFB gene locus, the endogenous CFB gene nucleotide sequence is replaced with a nucleotide sequence comprising a nucleotide sequence of the human CFB gene.

9. The animal of claim 8, wherein the expression of the nucleotide sequence of the human CFB gene is regulated by a regulatory element, and the regulatory element is an endogenous regulatory element or a human regulatory element;
preferably, the animal has one or more cells expressing human or chimeric CFB.

10. The animal of claim 8, wherein the animal does not express endogenous CFB or expresses a decreased level of endogenous CFB as compared to CFB expression level in a wild-type animal.

11. The animal of any one of claims 8-10, wherein the nucleotide sequence of the human CFB gene comprises a portion of exon 1 to a portion of exon 18 of the human CFB gene; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene.

12. The animal of claim 11, wherein the nucleotide sequence of human CFB gene further comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene.

13. The animal of any one of claims 8-12, wherein the nucleotide sequence of human CFB gene comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof.

14. The animal of any one of claims 8-13, wherein the nucleotide sequence of human CFB comprises a nucleotide sequence set forth in SEQ ID NO: 7 or 39, or a nucleotide sequence that is at least 70%, 75%, 80%, 85%, 90%, 95%, or 99% identical to SEQ ID NO: 7 or 39, or a nucleotide sequence that differs from SEQ ID NO: 7 or 39 by no more than 50, 40, 30, 20, 10, 9, 6, 3, or 1 nucleotide, or c a nucleotide sequence comprising a substitution, a deletion and/or insertion of one or more nucleotides set forth in SEQ ID NO: 7 or 39.

15. The animal of any one of claims 8-14, wherein the endogenous CFB gene exons 2 to 17 are replaced; preferably, the endogenous CFB gene further comprises a portion of exon 1, all of intron 1, and/or a portion of intron 17 are replaced; further preferably, the endogenous CFB gene further comprises a portion of exon 18 are replaced.

16. The animal of any one of claims 8-14, wherein the endogenous CFB gene is replaced from the start codon to the stop codon thereof, or the endogenous CFB gene is replaced from a portion of exon 1 to a portion of intron 17, wherein the portion of exon 1 comprises up to 70 nucleotides at the 3' end of exon 1.

17. The animal of any one of claims 8-16, wherein the animal is a mammal, such as a monkey or a rodent (e.g., a mouse or a rat).

18. The animal of any one of claims 8-17, wherein the animal whose genome comprises a modified endogenous CFB nucleotide sequence that transcribes an mRNA comprising SEQ ID NO: 8 or 40, or a nucleotide sequence that is at least 70%, 75%, 80%, 85%, 90%, 95%, or 99% identical to SEQ ID NO: 8 or 40, or a nucleotide sequence that differs from SEQ ID NO: 8 or 40 by no more than 50, 40, 30, 20, 10, 9, 6, 3, or 1 nucleotide, or a nucleotide sequence comprising a substitution, a deletion and/or insertion of one or more nucleotides set forth in SEQ ID NO: 8 or 40.

19. The animal of claim 8, wherein the animal is homozygous or heterozygous with respect to the replacement at the endogenous CFB gene locus.

20. The animal of any one of claims 1-19, wherein the animal expresses the human or chimeric CFB having at least one mouse CFB activity and/or at least one human CFB activity.

21. The animal of any one of claims 1-20, wherein the animal further comprises a sequence encoding an additional human or chimeric protein, and the additional human or chimeric protein is one or more selected from the group consisting of CFD, C3, C5, C5AR1, ANGPTL3, IL36R, IGF1R, TSLP, TLR8, OX40, PD-1, PD-L1, and CTLA4.

22. A non-human animal genome, wherein the animal genome comprises at least one chromosome comprising a sequence encoding a human or chimeric complement factor B.

23. The animal genome of claim 22, wherein at the endogenous CFB gene locus, the endogenous CFB gene nucleotide sequence is replaced with a nucleotide sequence comprising a nucleotide sequence of the human CFB gene.

24. The animal genome of claim 23, wherein the nucleotide sequence of the human CFB gene comprises a portion of exon 1 to a portion of exon 18 of the human CFB gene; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene.

25. The animal genome of claim 23, wherein the nucleotide sequence of human CFB gene further comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene.

26. The animal genome of 23, wherein the nucleotide sequence of human CFB gene comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof.

27. The animal genome of any one of claims 23-26, wherein the endogenous CFB gene exons 2 to 17 are replaced; preferably, the endogenous CFB gene further comprises a portion of exon 1, all of intron 1, and/or a portion of intron 17 are replaced; further preferably, the endogenous CFB gene further comprises a portion of exon 18 are replaced.

28. The animal genome of claims 27, wherein the endogenous CFB gene is replaced from the start codon to the stop codon thereof, or the endogenous CFB gene is replaced from a portion of exon 1 to a portion of intron 17, wherein the portion of exon 1 comprises up to 70 nucleotides at the 3' end of exon 1.

29. A method for making a genetically-modified, non-human animal, at the endogenous CFB gene locus in at least one cell of the non-human animal, the endogenous CFB gene nucleotide sequence is replaced with a nucleotide sequence comprising a nucleotide sequence of the human CFB gene.

30. The method of claims 29, wherein the nucleotide sequence of the human CFB gene comprises a portion of exon 1 to a portion of exon 18 of the human CFB gene; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene.

31. The method of claims 30, wherein the nucleotide sequence of human CFB gene further comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene.

32. The method of any one of claims 29-31, wherein the nucleotide sequence of human CFB gene comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof.

33. A method of making a genetically-modified non-human animal cell that expresses a human or chimeric CFB the method comprising: at an endogenous CFB gene locus, the endogenous CFB gene nucleotide sequence is replaced with a nucleotide sequence comprising a nucleotide sequence of the human CFB gene.

34. The method of claim 33, wherein the nucleotide sequence of the human CFB gene comprises a portion of exon 1 to a portion of exon 18 of the human CFB gene; preferably, the nucleotide sequence of the human CFB gene comprises a nucleotide sequence of the coding region of the human CFB gene; further preferably, the nucleotide sequence of the human CFB gene comprises a nucleotide sequence from the start codon to the stop codon of the human CFB gene.

35. The method of claim 34, wherein the nucleotide sequence of human CFB gene further comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene. preferably, the nucleotide sequence of human CFB gene comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof.

36. The method of any one of claims 33-35, wherein the expression of the nucleotide sequence of the human CFB gene is regulated by a regulatory element, and the regulatory element is an endogenous regulatory element or a human regulatory element.

37. A humanized CFB gene comprising a nucleotide sequence, wherein the nucleotide sequence is one of the following:
a) a nucleotide sequence encoding SEQ ID NO: 2 or amino acids 26-764 of SEQ ID NO: 2;
b) SEQ ID NO: 3, 4, 5, 6, 7, 8, 9, 10, 11, 12, 37, 38, 39, 40, 41, 42, 43, 44, 45, or 46;
c) a nucleotide sequence having at least 90% identity to SEQ ID NO: 3, 4, 5, 6, 7, 8, 9, 10, 11, 12, 37, 38, 39, 40, 41, 42, 43, 44, 45, or 46;
d) a nucleotide sequence is at least 91%, 92%, 93%, 94%, 95%, 96%, 97%, 98%, or 99% identity to SEQ ID NO: 3, 4, 5, 6, 7, 8, 9, 10, 11, 12, 37, 38, 39, 40, 41, 42, 43, 44, 45, or 46.

38. A cell, tissue, or organ, wherein the cell, tissue, or organ comprises the humanized CFB gene of claim 37.

39. An animal model, wherein the animal comprises the humanized CFB gene of claim 37 or the cell, tissue, or organ of claim 38.

40. A targeting vector, wherein the targeting vector comprises a 5' arm, a donor region, and a 3' arm, wherein the 5' arm is homologous to the 5' end of a region to be altered, the 3' arm is homologous to the 3' end of the region to be altered, and the donor region comprises a nucleotide sequence encoding a human or chimeric CFB.

41. The targeting vector of claim 40, wherein the region to be altered is located at the endogenous CFB gene locus of a non-human animal; preferably, the region to be altered comprises at least one exon or at least one intron of the endogenous CFB gene of the non-human animal, e.g., a portion of exon 1 to a portion of intron 17 of the endogenous CFB gene, or a portion of exon 1 to a portion of exon 18 of the endogenous CFB gene.

42. The targeting vector of claim 40, wherein the donor region comprises a portion of exon 1 to a portion of exon 18 of the human CFB gene; preferably, the donor region comprises a nucleotide sequence of the coding region of the human CFB gene; further preferably, the donor region comprises a nucleotide sequence from the start codon to the stop codon of the human CFB gene.

43. The targeting vector of claim 42, wherein t the donor region comprises the 5'UTR and/or the 3'UTR of human CFB gene, and further comprises at least a 50 bp contiguous nucleotide sequence upstream of the 5'UTR of human CFB gene and/or at least a 50 bp contiguous nucleotide sequence downstream of the 3'UTR of human CFB gene.
preferably, the donor region comprises the 5'UTR of human CFB and at least a 50 bp contiguous nucleotide sequence upstream thereof, a nucleotide sequence from the start codon to the stop codon of the human CFB gene, and the 3'UTR of human CFB and at least a 50 bp contiguous nucleotide sequence downstream thereof.

44. A use of the non-human animal of any one of claims 1-21, the non-human animal genome of any one of claims 22-28, a non-human animal obtained by the making method of any one of claims 29-32, a cell obtained by the making method of any one of claims 33-36, the humanized CFB gene of claim 37, the cell, tissue, or organ of claim 38, the animal model of claim 39, or the targeting vector of any one of claims 40-43, wherein the use comprises:
a) use in the development of products related to CFB-associated immune processes involving human cells;
b) use as a model system for CFB-associated pharmacological, immunological, microbiological, and medical research;
c) use in the production and utilization of animal experimental disease models for CFB-associated etiological studies and/or for the development of diagnostic strategies and/or for the development of therapeutic strategies;
d) use in the *in vivo* screening of human CFB signaling pathway modulators, pharmacological efficacy detection, efficacy evaluation, validation, or assessment; or,
e) use in the study of CFB gene function, the study of drugs targeting human CFB targets, pharmacological efficacy, and the study of drugs for CFB-associated inflammatory and immune-related diseases.

45. A method of determining the effectiveness of a CFB therapeutic agent for treating a disease, wherein the method comprises:
1) administering a CFB therapeutic agent to the animal of any one of claims 1-21, a animal obtained by the making method of any one of claims 29-32, or the animal model of claim 39, wherein the non-human animal has a disease; and
2) determining the effects of the CFB therapeutic agent in treating the disease;
preferably, the disease is an immune disease, inflammation, or a tumor.

46. The method of claim 45, wherein the immune disease is one or more selected from the group consisting of age-related macular degeneration (AMD), rheumatoid arthritis, colitis (including ulcerative colitis or Crohn's disease, e.g., perianal Crohn's disease), rheumatism, multiple sclerosis, Parkinson's disease, asthma, myasthenia gravis, and complement diseases.

47. The method of claim 45, wherein the inflammation is one or more selected from the group consisting of IgA nephropathy, C3 glomerulopathy, glomerulonephritis, degenerative inflammation, exudative inflammation (e.g., serous inflammation, fibrinous inflammation, purulent inflammation, hemorrhagic inflammation, necrotizing inflammation, or catarrhal inflammation), proliferative inflammation, and specific inflammation (e.g., tuberculosis, syphilis, leprosy, or lymphogranuloma).

48. The method of claim 45, wherein the tumor is solid tumors (e.g., breast cancer) or blood cell tumors (e.g., lymphocyte tumors, B- or T-cell tumors).

49. A method of determining toxicity of a CFB therapeutic agent comprising:
1) administering the CFB therapeutic agent to the non-human animal of any one of claims 1-21, a non-human animal obtained by the making method of any one of claims 29-32, or the animal model of claim 39;
2) determining effects of the CFB therapeutic agent to the animal.

50. The method of claim 49, wherein determining effects of the CFB therapeutic agent to the animal involves measuring the body weight or performing a blood test, preferably, the blood test comprises one or more selected from the group consisting of red blood cell count, hematocrit, and hemoglobin.
